# EUROPEAN PATENT APPLICATION

(11) **EP 3 195 868 A2**
(43) Date of publication of application: **26.07.2017**
(21) Application number: 16199830.7
(22) Date of filing: 03.10.2011
(51) Int. Cl.: A61K 31/55, A61K 31/122, A61K 9/127, A61K 9/48, A61P 35/00, A61K 31/704, A61K 31/7068, A61K 9/00, A61K 31/675, A61K 45/06

(54) **THERAPEUTIC USE OF A TLR AGONIST AND COMBINATION THERAPY**

(30) Priority: 01.10.2010 US 388967 P; 01.10.2010 US 388953 P; 06.10.2010 US 390447 P
(62) Divisional of application: 11830073.0
(71) Applicant: VentiRx Pharmaceuticals, Inc., Seattle, WA 98101 (US); The Trustees of the University of Pennsylvania, Philadelphia, PA 19104-6283 (US)
(72) Inventor: HERSHBERG, Robert, Seattle, WA Washington 98101-1397 (US); COUKOS, George, Wynnewood, PA Pennsylvania 19096 (US); DIETSCH, Gregory, Snohomish, WA Washington 98296 (US); FACCIABENE, Andrea, Philadelphia, WA Washington 19130 (US); MANJARREZ, Kristi, Seattle, WA Washington (US); RANDALL, Tressa D., Mukilteo, WA Washington 98275 (US)
(74) Representative: Cooley (UK) LLP

(57) **Abstract**

The present invention is directed generally to formulations of a TLR agonist preferably a TLR8 agonist, and its use in the treatment of various diseases, including combination therapies for treating cancer.

## Description

### CROSS-REFERENCE TO RELATED APPLICATION

This application claims priority to, and the benefit of, U.S. provisional application No. 61/388,953, filed October 1, 2010, U.S. provisional application No. 61/388,967, filed October 1, 2010, and U.S. provisional application No. 61/390,447, filed October 6, 2010, the contents of which are incorporated herein by reference in their entireties.

### FIELD OF THE INVENTION

The present invention is directed to formulations of a TLR agonist, preferably a TLR8 agonist, and a combination therapy comprising administration of a TLR8 agonist and an anti-cancer agent for use in the treatment of cancer.

### BACKGROUND OF THE INVENTION

Stimulation of the immune system, which includes stimulation of either or both innate immunity and adaptive immunity, is a complex phenomenon that can result in either protective or adverse physiologic outcomes for the host. In recent years there has been increased interest in the mechanisms underlying innate immunity, which is believed to initiate and support adaptive immunity. This interest has been fueled in part by the recent discovery of a family of highly conserved pattern recognition receptor proteins known as Toll-like receptors (TLRs) believed to be involved in innate immunity as receptors for pathogen associated molecular patterns (PAMPs). Compositions and methods useful for modulating innate immunity are therefore of great interest, as they may affect therapeutic approaches to conditions involving cancer, infectious disease, autoimmunity, inflammation, allergy, asthma, graft rejection, graft versus host disease (GvHD), and immunodeficiency.

Toll-like receptors (TLRs) are a family of type I transmembrane proteins whose in vivo activation initiates an innate immune response involving specific cytokines, chemokines and growth factors. While all TLRs can activate certain intracellular signaling molecules such as nuclear factor kappa beta (NF-kB) and mitogen activated protein kinases (MAP kinases), the specific set of cytokines and chemokines released appears to be unique for each TLR. TLR7, 8, and 9 comprise a subfamily of TLRs which are located in endosomal or lysosomal compartments of immune cells such as dendritic cells and monocytes. In contrast to TLR7 and 9 which are highly expressed on plasmacytoid dendritic cells (pDC), TLR8 is mainly expressed on myeloid DC (mDC) and monocytes. This subfamily mediates recognition of microbial nucleic acids, such as single stranded RNA. Agonists of TLR8 stimulate the production of various inflammatory cytokines including interleukin-6, interleukin-12, tumor necrosis factor-alpha, and interferon-gamma. Such agonists also promote the increased expression of co-stimulatory molecules such as CD40, CD80, CD83, and CD86, major histocompatibility complex molecules, and chemokine receptors. The type I interferons, IFNα and IFNβ, are also produced by cells upon activation with TLR8 agonists.

Small, low-molecular weight (less than 400 Daltons) synthetic imidazoquinoline compounds which resemble the purine nucleotides adenosine and guanosine were the first TLR7 and TLR8 agonists to be identified. A number of these compounds have demonstrated anti-viral and anti-cancer properties. For example, the TLR7 agonist imiquimod (ALDARA™) was approved by the U.S. Food and Drug Administration as a topical agent for the treatment of skin lesions caused by certain strains of the human papillomavirus. Imiquimod may also be useful for the treatment of primary skin cancers and cutaneous tumors such as basal cell carcinomas, keratoacanthomas, actinic keratoses, and Bowen's disease. The TLR7/8 agonist resiquimod (R-848) is being evaluated as a topical agent for the treatment of human genital herpes.

Doxorubicin is a drug used in cancer chemotherapy. It is an anthracycline antibiotic, closely related to the natural product daunomycin, and like all anthracyclines it works by intercalating DNA. Doxorubicin is commonly used in the treatment of a wide range of cancers, including hematological malignancies, many types of carcinoma, and soft tissue sarcomas.

### SUMMARY OF THE INVENTION

The present invention is directed generally to a combination therapy comprising administration of a benzo[b]azepine TLR8 agonist and one or more additional treatment modalities such as an anti-cancer agent (e.g., doxorubicin) for use in treating, alleviating, or preventing cancer, preferably solid tumors (such as sarcomas, carcinomas, and lymphomas), and for other uses including the treatment of leukemias, the treatment of certain skin conditions or diseases, such as atopic dermatitis, the treatment of infectious diseases, preferably viral diseases, and for use as adjuvants in vaccines formulated for use in cancer therapy and in the treatment of infectious diseases. Specifically, the present invention is directed to methods and compositions comprising a benzo[b]azepine TLR8 agonist, VTX-2337, and doxorubicin. In preferred embodiments, VTX-2337 and doxorubicin are used for the treatment of cancer and the cancer is selected from the group consisting of ovarian cancer, breast cancer, head and neck cancer, renal cancer, bladder cancer, hepatocellular cancer, colorectal cancer, melanoma, and lymphoma, or any combination thereof.

Preferably, VTX-2337 is formulated at a concentration of from about 0.001 mg/ml to about 50 mg/ml, from about 0.01 mg/ml to about 50 mg/ml, from about 0.5 mg/ml to about 50 mg/ml, from about 1 mg/ml to about 40 mg/ml, or from about 2 mg/ml to about 15 mg/ml. In certain embodiments, VTX-2337 is formulated at a concentration of from about 0.5 mg/ml to about 10 mg/ml, from about 0.5 mg/ml to about 8 mg/ml, from about 0.5 mg/ml to about 6 mg/ml, from about 0.5 mg/ml to about 4 mg/ml, or from about 0.5 mg/ml to about 2 mg/ml. In certain embodiments, VTX-2337 is formulated at a concentration of about 0.5 mg/ml, about 1 mg/ml, about 2 mg/ml, about 4 mg/ml, about 6 mg/ml, about 8 mg/ml, about 10 mg/ml, about 15 mg/ml, about 20 mg/ml, about 25 mg/ml, about 30 mg/ml, about 40 mg/ml, or about 50 mg/ml. Preferably, the formulation comprises about 1-30%, 5-15%, or 5-10% weight/volume (w/v) of a cyclodextrin, preferably a β-cyclodextrin, and most preferably sulfobutylether β-cyclodextrin. In certain embodiments, the formulation comprises 1%, 5%, 10%, 15%, 20%, 25%, or 30% w/v of a cyclodextrin, preferably a β-cyclodextrin, and most preferably sulfobutylether β-cyclodextrin. In a particular embodiment, the formulation is an aqueous solution comprising VTX-2337 at a concentration of at least 2 mg/ml. In a further embodiment, the formulation comprises 15% w/v of a cyclodextrin, preferably a β-cyclodextrin, and most preferably sulfobutyl ether β-cyclodextrin. In preferred embodiments, the formulation is suitable for injection in a mammal, preferably a human. In particular embodiments, injection is by a subcutaneous route, an intramuscular route, or transdermal route. In certain embodiments, the formulation is suitable for intravenous administration.

Preferably, the reconstituted formulation is suitable for injection in a mammal, preferably a human. In particular embodiments, injection is by a subcutaneous route, an intramuscular route, or transdermal route. In certain embodiments, the formulation is suitable for intravenous administration.

The present invention further provides methods for the treatment of cancer by administering to a subject, preferably a human subject, doxorubicin and TLR8 agonist VTX-2337, which contains a cyclodextrin. In a preferred embodiment, VTX-2337 is administered in combination with one or more additional treatment modalities, where the modalities are selected from a chemotherapeutic agent, a cytokine, an antibody, hormonal therapy, or radiation therapy. In one embodiment, VTX-2337 is administered as part of a regimen for the treatment of a solid tumor. In a further embodiment, the solid tumor is a form of cancer selected from among ovarian cancer, breast cancer, head and neck cancer, renal cancer, bladder cancer, hepatocellular cancer, colorectal cancer, or lymphoma, or any combination thereof. In one embodiment, VTX-2337 is administered as part of a regimen for the treatment of a lymphoma. In one embodiment, the lymphoma is Hodgkin's lymphoma. In another embodiment, the lymphoma is non-Hodgkin's lymphoma. In another embodiment, VTX-2337 is used as a vaccine adjuvant for the treatment of cancer. In certain embodiments of the methods for the treatment of cancer, VTX-2337 is administered by injection or intravenously. In particular embodiments, injection is by a subcutaneous route, an intramuscular route, or a transdermal route. In a particular embodiment, the formulation is administered by subcutaneous injection.

In certain embodiments of the methods for treating cancer, VTX-2337 is administered to the subject at a dose of about 0.02 to 10 mg/kg (e.g., about 0.05-0.075 mg/kg, or about 0.04 to 5 mg/kg) body weight of the subject. In certain embodiments, VTX-2337 is administered at a dose of about .02 mg/kg, about 0.05 mg/kg, about 1 mg/kg, about 2 mg/kg, or about 5 mg/kg. For example, assuming the subject has a body weight of about 70 kg, VTX-2337 is administered at a dose of about 1.4 mg-700 mg (e.g., 3.5 mg-5.25 mg, or about 2.8-350 mg). In certain further embodiments, VTX-2337 is administered to the subject on a weekly or biweekly basis.

The present invention also provides a pharmaceutical pack or kit comprising one or more containers filled with a liquid or lyophilized VTX-2337 and an anti-cancer agent (e.g., doxorubicin) of the invention for the treatment of cancer or one or more symptoms thereof. The liquid or lyophilized VTX-2337 and an anti-cancer agent (e.g., doxorubicin) can be packed in the same of different containers of the kit. Preferably, the formulation of VTX-2337 comprises about 1-30%, 5-15%, or 5-10% w/v of a cyclodextrin, preferably a β-cyclodextrin, and most preferably sulfobutylether β-cyclodextrin. In certain embodiments, the formulation VTX-2337 comprises 2%, 5%, 10%, 15%, 20%, 25%, or 30% w/v of a cyclodextrin, preferably a β-cyclodextrin, and most preferably sulfobutylether β-cyclodextrin. In a particular embodiment, the formulation is an aqueous formulation of VTX-2337. Preferably, VTX-2337 is formulated at a concentration of at least 2 mg/ml and the formulation, whether aqueous or a reconstituted lyophilized formulation, is suitable for subcutaneous injection in a mammal, preferably a human.

In one embodiment, VTX-2337 is formulated at a concentration of at least 2 mg/ml. Moreover, the formulation is suitable for administration to the subject, where the subject is preferably a human, by injection and is by subcutaneous, intramuscular, or transdermal injection. In certain embodiments, VTX-2337 is administered to the subject at a dose of about 0.02 to 10 mg/kg, at a dose of about 0.04 to 5 mg/kg or at a dose of about 0.05-0.075 mg/kg. In certain further embodiments, VTX-2337 is administered to the subject on a weekly or biweekly basis.

In a preferred embodiment, VTX-2337 is administered in combination with one or more additional treatment modalities, where the modalities are selected from a chemotherapeutic agent, a cytokine, an antibody, hormonal therapy, or radiation therapy. The present invention also provides methods for the treatment of infectious disease is caused by a virus, where the virus is a hepatitis virus.

In a preferred embodiment, doxorubicin is formulated for injection, most preferably intravenous administration. In certain embodiments, VTX-2337 of the invention is formulated for administration by an intradermal, a transdermal, a subcutaneous, or an intramuscular route.

In certain embodiments of the methods for treating cancer, doxorubicin is administered to the subject at a dose of from about 0.02 to 10 mg/kg of body weight or about 0.04 to 5 mg/kg of body weight of the subject.

The present invention also provides a method of treating cancer with a low-dose formulation of a benzo[b]azepine TLR8 agonist. The method comprising administering to a subject in need thereof a benzo[b]azepine TLR8 agonist at a dose below 0.007 mg/kg/week, e.g., between 0.002 mg/kg/week to 0.006 mg/kg/week. In one embodiment, the benzo[b]azepine TLR8 agonist is 2-amino-N,N-dipropyl-8-(4-(pyrrolidine-1-carbonyl)phenyl)-3H-benzo[b]azepine-4-carboxamide. The method may include administering benzo[b]azepine TLR8 agonist as the only active ingredient or further include administering a second therapeutic agent such as an anti-cancer drug in combination with the low-dose formulation of the benzo[b]azepine TLR8 agonist. The second therapeutic agent can be another benzo[b]azepine TLR8 agonist or a drug molecule disclosed herein (e.g., doxorubicin, gemcitabine, or cyclophosphamide). The method can also be carried out in combination with one or more additional treatment modalities (e.g., radiation therapy) in a regiment for the treatment of cancer.

In another aspect, the invention also provides a subcutaneous dosage form comprising a benzo[b]azepine TLR8 agonist for the treatment of cancer in a subject, wherein the subcutaneous dosage form, upon administration to a human at a dosage of 2-4 mg/m² of the agonist, provides an AUC_{0-inf} of the agonist of about 55 to about 90 h*ng/mL, e.g., about 60 to about 80 h*ng/mL.

In yet another aspect, the invention also provides a subcutaneous dosage form comprising a benzo[b]azepine TLR8 agonist for the treatment of cancer in a subject, wherein the subcutaneous dosage form, upon administration to a human at a dosage of 2-4 mg/m² of the agonist, provides a Cₘₐₓ of the agonist of about 10 to about 30 ng/mL, e.g., about 15 to about 25 ng/mL.

The invention also provides a pharmaceutical composition including a liquid or lyophilized formulation of benzo[b]azepine TLR8 agonist (e.g.,VTX-2337) and an anti-cancer agent (e.g., doxorubicin). The formulation of the agonist and the anti-cancer agent can be in the same pharmaceutical composition or in different compositions, in which case, the formulation of the agonist and the anti-cancer agent can be administered concurrently or sequentially.

The above description sets forth rather broadly the more important features of the present invention in order that the detailed description thereof that follows may be understood, and in order that the present contributions to the art may be better appreciated. Other objects and features of the present invention will become apparent from the following detailed description considered in conjunction with the examples.

### DESCRIPTION OF THE FIGURES

Figure 1A is a set of FACS images acquired on hematolymphoid cells from NSG-HIS mice. NSG mice received cord blood derived human hematopoietic CD34+ stem cells. Level of human cell engraftment (CD45+, CD45+CD14+, CD45+CD33) is shown. Mice were treated with 0.5 or 5 mg/kg of VTX-2337. Maturation of CD14+ cells (CD83, CD86) is shown.
Figure 1B is a set of bar graphs showing a change in the level of activation markers (CD86⁺, MHC Class II) on monocytes (CD45⁺ CD14⁺), mDC (CD45⁺ CD11c⁺) and pDC (CD45⁺ CD123⁺) 6 hours after SC administration of VTX-2337 to NSG-HIS mice.
Figure 1C is a set of bar graphs showing a change in plasma cytokine levels (INF-g, TNF-alpha, IL-12, and IL-10) 6 hours after SC administration of VTX-2337 to NSG-HIS mice.
Figure 2 is a set of bar graphs showing a change in plasma cytokine levels (IFN-g, IL-10, TNF-alpha) in mice that received no treatment (CTRL), Doxil at the maximum tolerated dose (MTD, 50 mg/m²) or 5 mg/kg VTX-2337 5 days after treatment with Doxil.
Figure 3A is a schematic showing the protocol for treating NSG-HIS mice with Doxil, VTX-2337 or their combination in a humanized mouse (NSG-HIS) ovarian cancer model that used the human ovarian cancer cell line OVCAR5 to generate tumors.
Figure 3B is a line graph showing size changes in tumors of NSG-HIS mice treated with Doxil at 50 mg/m², VTX-2337 at 0.5mg/kg, or their combination over time after inoculation with OVCAR5 cells.
Figure 3C is a set of IHC images showing tumors infiltrated with CD45⁺ cells from mice treated with Doxil at 50 mg/m², VTX-2337 at 0.5mg/kg, or their combination in a humanized ovarian cancer model.
Figure 3D is a set of bar graphs showing a change in the level of tumor-infiltrating CD3⁺, CD8⁺, CD69⁺ activated CD3⁺CD8⁺ T cells, and CD40⁺ activated macrophages (CD45⁺CD11b⁺), pDC (CD45⁺CD123⁺), and mDC (CD45⁺CD11c⁺) in mice treated with Doxil at 50 mg/m², VTX-2337 at 0.5mg/kg, or their combination in a humanized ovarian cancer model.
Figure 4A is a line graph showing changes in counts per minute (cpm) of lytic ⁵¹Cr labeled OVCAR5 cells lysed by the TIL, expanded from mice treated with Doxil or the combination of VTX-2337 and Doxil, in response to a varying ratio of effector TIL over target OVCAR5 cells.
Figure 4B is a line graph showing changes in the tumor size over time in NSG-HIS mice that were inoculated with OVCAR 5 cells and the treated with adoptively transferred TILS from mice in the experiment described in Figure 3.
Figure 4C is a set of line graphs showing changes in counts of lytic ⁵¹Cr labeled OVCAR5 cells lysed by TIL from the mice treated with Doxil or the combination of VTX-2337 and Doxil, in the absence or presence of an anti MHC class I (MHCI) neutralizing antibody.
Figure 4D is a set of bar graphs showing a change in the level of IFNg released by TIL incubated with OVCAR5 cells or melanoma cells.
Figure 5A is a bar graph showing a change in the percentage of apoptotic cells stained by annexin-V and 7AAD in OVCAR5 cells treated with conditioned media from human PBMCs that had been incubated with buffer alone, CD3/CD28 beads, or 1 µg/mL VTX-2337.
Figure 5B is a bar graph showing a change in the number of live cells in OVCAR5 cells treated with conditioned media from human PBMCs that had been incubated with buffer alone, CD3/CD28 beads, or 1 ug/mL VTX-2337.
Figure 5C is an image showing a western blot film showing TNFalpha-receptor 1 expression on OVCAR5 cells.
Figure 5D is a set of graphs showing FACS images of OVCAR5 cells treated with TNFalpha (10 ng/ml) or Doxil (1 µg/ml) or their combination and the resulting change in the percentage of apoptotic cells stained by annexin-V and 7AAD.
Figure 5E is an image showing a western blot film of FLIP_{L} expression on OVCAR 5 cells treated with 0.5 or 2.5 µg/mL Doxil.
Figure 5F is a set of graphs showing FACS images of OVCAR5 cells which were pre-cultured with 10 µg/ml cycloheximide (cyclx) for 24h and then treated with 10 or 50 ng/ml of TNFalpha, and a resulted change in the percentage of apoptotic cells stained by annexin-V and 7AAD.
Figure 6 is a set of graphs showing the potency and selectivity of VTX-2337 in peripheral blood mononuclear cells (PBMCs) from 15 healthy donors and also in HEK293 cells transfected with TLR8 or TLR7 and an NF-κB driven reporter gene.
Figure 7 is a set of graphs showing that VTX-2337 stimulates a range of cytokines and chemokines in human whole blood.
Figure 8 is a set of graphs showing that VTX-2337 activates monocytes and myeloid dendritic cells (mDCs) but not plasmacytoid dendritic cells (pDCs).
Figure 9 is a graph showing pharmacokinetics of VTX-2337 following subcutaneous administration. Numerical labels "1-8" in this Figure correspond to Cohorts 1-8 respectively.
Figures 10A and 10B are graphs showing consistent pharmacodynamic responses over multiple treatment cycles.

### DETAILED DESCRIPTION OF THE INVENTION

The details of one or more embodiments of the invention are set forth in the accompanying description below. Although any methods and materials similar or equivalent to those described herein can be used in the practice or testing of the present invention, the preferred methods and materials are now described. Other features, objects, and advantages of the invention will be apparent from the description. In the specification, the singular forms also include the plural unless the context clearly dictates otherwise. Unless defined otherwise, all technical and scientific terms used herein have the same meaning as commonly understood by one of ordinary skill in the art to which this invention belongs. In the case of conflict, the present specification will control.

The present invention provides composition and methods of using benzo[b]azepine TLR8 agonist, (e.g., VTX-2337) and another therapeutic modality (such as an anticancer agent, e.g., doxorubicin) to treat, alleviate, or prevent cancer or other disorders disclosed herein. VTX-2337 is a novel, potent and selective small molecule TLR8 agonist. Formulations of VTX-2337 are described in PCT International Publication WO10/014913. The formulations of the present invention are suitable for use in methods for the treatment of a human cancer as described herein.

Unless otherwise indicated, it is to be understood that the terminology used herein is for the purpose of describing particular embodiments only and is not intended to be limiting. In this specification and in the claims that follow, reference will be made to a number of terms, which shall be defined to have the definitions set forth below.

A "subject" in the context of the present invention is preferably a mammal. The mammal can be a human, non-human primate, mouse, rat, dog, cat, horse, or cow, but are not limited to these examples. A subject can be male or female. A subject can be one who has been previously diagnosed or identified as having cancer, and optionally has already undergone, or is undergoing, a therapeutic intervention for the cancer such as Doxil treatment or radiation therapy. Alternatively, a subject can also be one who has not been previously diagnosed as having cancer, but who is at risk of developing such condition. For example, a subject can be one who exhibits one or more symptoms for cancer.

The terms "disease," "disorder" and "condition" are used interchangeably herein, and refer to any disruption of normal body function, or the appearance of any type of pathology. The etiological agent causing the disruption of normal physiology may or may not be known. Furthermore, although two patients may be diagnosed with the same disorder, the particular symptoms displayed by those individuals may or may not be identical.

The terms "treating" and "treatment" as used herein refer to reduction in severity and/or frequency of symptoms, elimination of symptoms and/or underlying cause, prevention of the occurrence of symptoms and/or their underlying cause, and improvement or remediation of damage. For example, treatment of a patient by administration of an anti-cancer agent of the invention encompasses chemoprevention in a patient susceptible to developing cancer (e.g., at a higher risk, as a result of genetic predisposition, environmental factors, or the like) and/or in cancer survivors at risk of cancer recurrence, as well as treatment of a cancer patient dual by inhibiting or causing regression of a disorder or disease.

The term "alleviating" or "ameliorating" as used herein refers to alleviate of at least one symptom of the disease, disorder, or condition.

The term "preventing" as used herein includes either preventing or slowing the onset of a clinically evident disease progression altogether or preventing or slowing the onset of a preclinical evident stage of a disease in individuals at risk. This includes prophylactic treatment of those at risk of developing a disease.

When referring to a compound of the invention, applicants intend the term "compound" to encompass not only the specified molecular entity but also its pharmaceutically acceptable, pharmacologically active analogs, including, but not limited to, salts, esters, amides, prodrugs, conjugates, active metabolites, and other such derivatives, analogs, and related compounds.

By the terms "effective amount" and "therapeutically effective amount" of a compound of the invention is meant a nontoxic but sufficient amount of the drug or agent to provide the desired effect.

By "pharmaceutically acceptable" is meant a material that is not biologically or otherwise undesirable, i.e., the material may be incorporated into a pharmaceutical composition administered to a patient without causing any undesirable biological effects or interacting in a deleterious manner with any of the other components of the composition in which it is contained. When the term "pharmaceutically acceptable" is used to refer to a pharmaceutical carrier or excipient, it is implied that the carrier or excipient has met the required standards of toxicological and manufacturing testing or that it is included on the Inactive Ingredient Guide prepared by the U.S. Food and Drug administration. "Pharmacologically active" (or simply "active") as in a "pharmacologically active" derivative or analog, refers to a derivative or analog having the same type of pharmacological activity as the parent compound and approximately equivalent in degree.

By "as-needed," as in "as-needed administration" or "in need thereof" is meant that a formulation is administered to a patient when symptoms are observed, or when symptoms are expected to appear, or at any time that the patient and/or treating physician deems it appropriate to treat (therapeutically or prophylactically) undesirable symptoms (e.g., symptoms arising from cancer).

### TLR Agonists of the Invention

### 1.1 Formulation

VTX-2337 formulations comprise an active compound with the following structure. The formulations of the present invention are suitable for subcutaneous administration to a subject, preferably a human subject, but can be used for administration by other means.

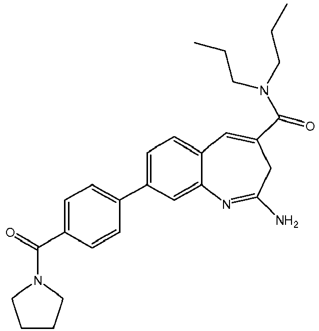

The VTX-2337 formulations of the present invention comprise one or more pharmaceutically acceptable excipients. The term excipient as used herein broadly refers to a biologically inactive substance used in combination with the active agents of the formulation. An excipient can be used, for example, as a solubilizing agent, a stabilizing agent, a diluent, an inert carrier, a preservative, a binder, a disintegrant, a coating agent, a flavoring agent, or a coloring agent. Preferably, at least one excipient is chosen to provide one or more beneficial physical properties to the formulation, such as increased stability and/or solubility of the active agent(s). VTX-2337 as described herein is the primary active agent in the formulations of the present invention. However, VTX-2337 may be formulated with other active agents, e.g., other TLR agonists, anti-cancer agents or anti-viral agents, as described herein.

A "pharmaceutically acceptable" excipient is one that has been approved by a state or federal regulatory agency for use in animals, and preferably for use in humans, or is listed in the U.S. Pharmacopia, the European Pharmacopia or another generally recognized pharmacopia for use in animals, and preferably for use in humans.

Examples of excipients include certain inert proteins such as albumins; hydrophilic polymers such as polyvinylpyrrolidone; amino acids such as aspartic acid (which may alternatively be referred to as aspartate), glutamic acid (which may alternatively be referred to as glutamate), lysine, arginine, glycine, and histidine; fatty acids and phospholipids such as alkyl sulfonates and caprylate; surfactants such as sodium dodecyl sulphate and polysorbate; nonionic surfactants such as such as TWEEN^{®}, PLURONICS^{®}, or polyethylene glycol (PEG); carbohydrates such as glucose, sucrose, mannose, maltose, trehalose, and dextrins, including cyclodextrins; polyols such as mannitol and sorbitol; chelating agents such as EDTA; and salt-forming counter-ions such as sodium.

The formulations of VTX-2337 may contain a cyclodextrin which increases the aqueous solubility of the TLR agonist. Cyclodextrins are crystalline, nonhygroscopic cyclic oligomers of α-D-glucopyranose. As a result of a lack of rotation about the bonds connecting the glucopyranose units, the cyclodextrins are not cylindrical, but toroidal in shape. Because of this restricted rotation they have a rigid structure with a central cavity whose size varies according to the number of glucopyranose units in the molecule. The three most common cyclodextrins are α-cyclodextrin, β-cyclodextrin and γ-cyclodextrin, which consist of six, seven, or eight glucopyranose units, respectively. Due to the arrangement of hydroxyl groups within the cyclodextrin molecule and the shape of the molecule, the internal surface of the cavity is hydrophobic, while the outside surface is hydrophilic. The primary hydroxyl groups are located on the narrower (inner) side of the toroidal molecule, while the secondary hydroxyl groups are located on the wider (outer) edge. This arrangement permits the cyclodextrins to accommodate a wide variety of small hydrophobic molecules within the hydrophobic cavity by forming an inclusion complex.

Suitable cyclodextrins for use in the formulations of the invention are known in the art. For example, TRAPPSOL™ and other cyclodextrins are made by CTD, Inc. (High Springs, FL), and CAPTISOL^{®} (sulfobutylether β-cyclodextrin) is present in commercially available injectables such as ABILIFY IM™, GEODON, and VFEND IV. Preferably, CAPTISOL^{®} is used in the formulations of the present invention.

Other water-solubilizing agents may be used. Examples of other such agents include Poloxamer, Povidone K17, Povidone K12, Tween 80, ethanol, Cremophor/ethanol, polyethylene glycol 300, polyethylene glycol 400, and propylene glycol. In preferred embodiments, the formulations of the invention contain less than 10% v/v of such agents. In certain embodiments, oil-based solubilizing agents such as lipiodol or peanut oil, are used.

In certain embodiments, the formulations of VTX-2337 may be prepared as a liquid or in a solid form such as a powder, tablet, pill or capsule. Liquid formulations may take such forms as suspensions, solutions, or emulsions in oily or aqueous vehicles, and may contain formulatory agents such as suspending, stabilizing and/or dispersing agents. In one embodiment, the formulation is an aqueous solution. In another embodiment, the final formulation is lyophilized. In other embodiments, the formulation comprises a colloidal drug delivery system. Such drug delivery systems include, for example, liposomes, albumin microspheres, microemulsions, nanoparticles and nanocapsules.

In one embodiment, VTX-2337 is a liquid or lyophilized formulation suitable for injection in a mammal, preferably a human. In one embodiment, the formulation is sterile. In another embodiment, the formulation is a sterile lyophilized formulation which is suitable for injection upon reconstitution with an amount of an aqueous carrier. In one embodiment, the liquid or lyophilized formulation is prepared as a unit dosage form as described below. The formulations may or may not contain an added preservative.

In certain embodiments, VTX-2337 further comprises one or more adjuvants. Examples of suitable adjuvants include potentiators of the immune response such as microbial derivatives (e.g., bacterial products, toxins such as cholera toxin and heat labile toxin from E. coli, lipids, lipoproteins, nucleic acids, peptidogylcans, carbohydrates, peptides), cells, cytokines, (e.g., dendritic cells, IL-12, and GM-CSF), hormones, and small molecules. Adjuvants contemplated include, but are not limited to, oil-based adjuvants (e.g., Freund's adjuvant), CpG oligonucleotides, aluminum salt adjuvants, calcium salt adjuvants, emulsions and surfactant-based formulations (e.g., MF59, ASO2, montanide, ISA-51, ISA-720, and QA21).

According to certain embodiments, VTX-2337 is formulated at a concentration of from about 0.5 to about 50 mg/ml. In some embodiments, the benzo[b]azepine TLR agonist is formulated at a concentration of from about 1 mg/ml to about 5 mg/ml, from about 1 mg/ml to about 10 mg/ml, from about 1 mg/ml to about 20 mg/ml, or from about 1 mg/ml to about 30 mg/ml. In other embodiments, VTX-2337 is formulated at a concentration of from about 0.5 mg/ml to about 1 mg/ml, from about 0.5 mg/ml to about 2 mg/ml, or from about 0.5 mg/ml to about 5 mg/ml. In certain embodiments, VTX-2337 is formulated at a concentration of between 0.5 and 10 mg/ml, between 0.5 and 5 mg/ml, or between 1 and 5 mg/ml. In other embodiments, VTX-2337 is formulated at a concentration of between 10-20 mg/ml, 20-30 mg/ml, or between 30-50 mg/ml. In specific embodiments, VTX-2337 is formulated at a concentration of about 1 mg/ml, about 2 mg/ml, about 4 mg/ml, about 5 mg/ml, about 6 mg/ml, about 8 mg/ml, about 10 mg/ml, about 15 mg/ml, about 20 mg/ml, about 25 mg/ml, about 30 mg/ml, or about 40 mg/ml.

The formulations of VTX-2337 can optionally be prepared as unit dosage forms. "Unit dosage form" refers to physically discrete units suitable for the intended use, i.e., as a single administration to the subject to be treated. Each unit contains a predetermined quantity of the active agent(s) formulated with the appropriate pharmaceutically acceptable excipient(s). For example, a unit dosage per vial may contain a certain volume, such as 1 ml, 2 ml, 3 ml, 4 ml, 5 ml, 6 ml, 7 ml, 8 ml, 9 ml, 10 ml, 15 ml, or 20 ml, having a particular concentration of the active agent. A dosage unit may comprise a single active agent, *i.e*., VTX-2337 as described herein, its derivatives and analogs, or mixtures thereof with other active agents (e.g., an anti-cancer agent such as doxorubicin) for use in combination therapies. In preferred embodiments, the unit dosage form comprises about 15 mg/ml to about 40 mg/ml of VTX-2337. The formulations are optionally contained in unit-dose or multi-dose containers, for example, in sealed ampules or vials, and may be in a lyophilized condition. Extemporaneous injection solutions and suspensions may be prepared from sterile powders, granules and tablets according to art-recognized methods. Examples of unit dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (e.g., nasal sprays or inhalers); gels; liquid dosage forms suitable for oral or mucosal administration to a patient, including suspensions (e.g., aqueous or non aqueous liquid suspensions, oil in water emulsions, or a water in oil liquid emulsions), solutions, and elixirs; liquid dosage forms suitable for subcutaneous administration to a subject; and sterile solids (e.g., crystalline or amorphous solids) that can be reconstituted to provide liquid dosage forms suitable for subcutaneous administration to a subject.

Additional information with regard to the methods of making the compositions and formulations and the ingredients comprising the compositions and formulations in accordance with the present invention can be found in standard references in the field, such as for example, "Remington's Pharmaceutical Sciences," Mack Publishing Co., Easton, PA.

### 1.2 Methods of Use

The combination of VTX-2337 and one or more additional treatment modalities (e.g., anti-cancer agents such as doxorubicin) is useful in methods for the treatment of cancer. Preferably, VTX-2337 formulations are used in combination with one or more additional treatment modalities in a regiment for the treatment of cancer. In certain embodiments, the cancer is a solid tumor. In one embodiment, the cancer is selected from the group consisting of ovarian cancer, breast cancer, head and neck cancer, renal cancer, bladder cancer, hepatocellular cancer, colorectal cancer, melanoma, and lymphoma, or any combination thereof. In a particular embodiment, the cancer is a lymphoma. In one embodiment, the lymphoma is non-Hodgkin's lymphoma.

Methods of testing efficacy of TLR8 agonist for treating cancer or the combination of an anti-cancer agent and a TLR8 agonist for treating cancer, include, but are not limited to *in vitro* assays such as those using human PBMC, HEK cells, or IHC and FACS for infiltrating cells, and lysis of tumor cells, and *in vivo* assays such as those using a NSG-HIS mice or humanized mouse (NSG-HIS) injected with ovarian cell lines, or human patients.

### 1.2.1 Combination Therapy

Combination therapy encompasses, in addition to the administration of VTX-2337, the adjunctive use of one or more modalities that aid in the prevention or treatment of cancer. Such modalities include, but are not limited to, chemotherapeutic agents, immunotherapeutics, anti-angiogenic agents, cytokines, hormones, antibodies, polynucleotides, radiation and photodynamic therapeutic agents. In specific embodiments, combination therapy can be used to prevent the recurrence of cancer, inhibit metastasis, or inhibit the growth and/or spread of cancer or metastasis. As used herein, "in combination with" means that VTX-2337 formulation of the invention is administered as part of a treatment regimen that comprises one or more additional treatment modalities as described in more detail in the following sections.

In certain embodiments, VTX-2337 is administered prior to, concurrently with, or subsequent to the administration of the one or more other modalities. In certain embodiments, VTX-2337 is administered prior to or subsequent to (e.g., 5 days after) the administration of an anti-cancer agent (e.g., doxorubicin). In one embodiment, VTX-2337 is formulated with one or more other modalities. In another embodiment, the one or more other modalities are administered in a separate pharmaceutical composition. In accordance with this embodiment, the one or more other modalities may be administered to a subject by the same or different routes of administration as those used to administer VTX-2337.

### 1.2.1.1 Combination with doxorubicin

In certain embodiments, the formulation comprising VTX-2337 is administered in combination with doxorubicin. Preferably, doxorubicin is in a pegylated liposomal form. The chemical structure of doxorubicin is shown below:

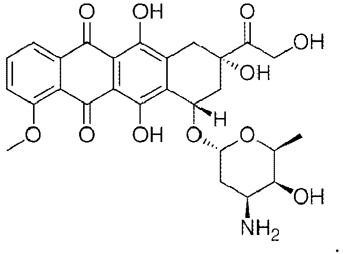

### 1.2.1.2 Combination with other anti-cancer agents

In certain embodiments, the formulation comprising VTX-2337 of the invention is administered in combination with one or more anti-cancer agents, preferably a chemotherapeutic agent. Such chemotherapeutic agents include, but are not limited to, the following groups of compounds: cytotoxic antibiotics, antimetabolities, anti-mitotic agents, alkylating agents, platinum compounds, arsenic compounds, DNA topoisomerase inhibitors, taxanes, nucleoside analogues, plant alkaloids, and toxins; and synthetic derivatives thereof. The following are non-limiting examples of particular compounds within these groups. Alkylating agents include nitrogen mustards such as cyclophosphamide, ifosfamide, trofosfamide, and chlorambucil; nitrosoureas such as carmustine (BCNU) and lomustine (CCNU); alkylsulphonates such as busulfan and treosulfan; and triazenes such as dacarbazine. Platinum containing compounds include cisplatin, carboplatin, aroplatin, and oxaliplatin. Plant alkaloids include vinca alkaloids such as vincristine, vinblastine, vindesine, and vinorelbine; and taxoids such as paclitaxel and docetaxol. DNA topoisomerase inhibitors include epipodophyllins such as etoposide, teniposide, topotecan, 9-aminocamptothecin, camptothecin, and crisnatol; and mitomycins such as mitomycin C. Anti-folates include DHFR inhibitors such as methotrexate and trimetrexate; IMP dehydrogenase inhibitors such as mycophenolic acid, tiazofurin, ribavirin, hydroxyurea and EICAR; and ribonuclotide reductase inhibitors such as deferoxamine. Pyrimidine analogs include uracil analogs such as 5-fluorouracil, floxuridine, doxifluridine, and ratitrexed; and cytosine analogs such as cytarabine (ara C), cytosine arabinoside, and fludarabine. Purine analogs include mercaptopurine and thioguanine. DNA antimetabolites include 3-HP, 2'-deoxy-5-fluorouridine, 5-HP, alpha-TGDR, aphidicolin glycinate, ara-C, 5-aza-2'-deoxycytidine, beta-TGDR, cyclocytidine, guanazole, inosine glycodialdehyde, macebecin II, and pyrazoloimidazole. Antimitotic agents include allocolchicine, halichondrin B, colchicine, colchicine derivative, dolstatin 10, maytansine, rhizoxin, thiocolchicine, and trityl cysteine.

Other examples of chemotherapeutic agents for use with the benzo[b]azepine TLR agonist formulations of the invention include isoprenylation inhibitors; dopaminergic neurotoxins such as 1-methyl-4-phenylpyridinium ion; cell cycle inhibitors such as staurosporine; actinomycins such as actinomycin D and dactinomycin; bleomycins such as bleomycin A2, bleomycin B2, and peplomycin; anthracyclines such as daunorubicin, doxorubicin (adriamycin), idarubicin, epirubicin, pirarubicin, zorubicin, and mitoxantrone; MDR inhibitors such as verapamil; and Ca²⁺ATPase inhibitors such as thapsigargin.

Compositions comprising one or more chemotherapeutic agents (*e.g*., FLAG, CHOP) are also contemplated for use in combination with VTX-2337 of the invention. FLAG comprises fludarabine, cytosine arabinoside (Ara-C) and G-CSF. CHOP comprises cyclophosphamide, vincristine, doxorubicin, and prednisone. Each of the foregoing lists is illustrative, and is not intended to be limiting.

In one embodiment, VTX-2337 is administered in combination with one or more of the following: IFNα, IL-2, Dacarbazine (Bayer), Temozolomide (Schering), Tamoxifen (AZ), Carmustine (BMS), Melphalan (GSK), Procarbazine (Sigma-Tau), Vinblastine, carboplatin, cisplatin, taxol, cyclophosphamide, doxorubin, Rituxan (Genentech/Roche), Herceptin (Genentech/Roche), Gleevec, Iressa (AZ), Avastin (Genentech/Roche), or Tarceva (Genentech/Roche).

In another embodiment, VTX-2337 of the invention is administered in combination with one or more of the following: an enediyne such as calicheamicin and esperamicin; duocarmycin, methotrexate, doxorubicin, melphalan, chlorambucil, Ara-C, vindesine, mitomycin C, cis-platinum, etoposide, bleomycin, and 5-fluorouracil.

Suitable toxins and chemotherapeutic agents that can be used in combination with the benzo[b]azepine TLR agonist formulations of this invention are described in Remington's Pharmaceutical Sciences, 19th Ed. (Mack Publishing Co. 1995), and in Goodman and Gilman's the Pharmacological Basis of Therapeutics, 7th Ed. (MacMillan Publishing Co. 1985). Other suitable toxins and/or chemotherapeutic agents are known to those of skill in the art.

Further examples of anti-cancer agents that can be used in combination with VTX-2337 of this invention include without limitation the following: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alfa-2a; interferon alfa-2b; interferon alfa-n1 ; interferon alfa-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinrosidine sulfate; vinzolidine sulfate; vorozole; zeniplatin; zinostatin; zorubicin hydrochloride.

Other anti-cancer agents that can be used include, but are not limited to: 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorlns; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-1 receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; lovastatin; loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anti-cancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; vitaxin; vorozole; zanoterone; zeniplatin; zilascorb; and zinostatin stimalamer.

### 1.2.1.3 Combination with radiation therapy

In another embodiment, VTX-2337 of the invention are administered in conjunction with a regimen of radiation therapy for the treatment of cancer. The methods encompass regimens comprising external-beam radiation therapy, interstitial implantation of radioisotopes (I-125, palladium, iridium), radioisotopes such as strontium-89, thoracic radiation therapy, intraperitoneal P-32 radiation therapy, and/or total abdominal and pelvic radiation therapy. Any suitable cytotoxic radionuclide or therapeutic isotope may be used in the regimen of radiation therapy. In certain embodiments, the isotope is an alpha-emitting isotope such as ²²⁵Ac, ²²⁴Ac, ²¹¹At, ²¹²Bi, ²¹³Bi, ²¹²Pb, ²²⁴Ra, or ²²³Ra. In other embodiments, the cytotoxic radionuclide is a beta-emitting isotope such as ¹⁸⁶Re, ¹⁸⁸Re, ⁹⁰Y, ¹³¹I, ⁶⁷Cu, ¹⁷⁷Lu, ¹⁵³Sm, ¹⁶⁶Ho, or ⁶⁴Cu. In some embodiments, cytotoxic radionuclide is an isotope that emits Auger and low energy electrons such as ¹²⁵I, ¹²³I or ⁷⁷Br. In other embodiments the isotope is ¹⁹⁸Au, ³²P, and the like.

In certain embodiments, the amount of the radionuclide administered to the subject is between about 0.001 mCi/kg and about 10 mCi/kg. In some embodiments, the amount of the radionuclide administered to the subject is between about 0.1 mCi/kg and about 1.0 mCi/kg. In other embodiments, the amount of the radionuclide administered to the subject is between about 0.005 mCi/kg and 0.1 mCi/kg.

### 1.2.1.4 Combination with therapeutic antibodies

In another embodiment, VTX-2337 of the invention is administered in combination with one or more immunotherapeutic agents, such as an antibody or a vaccine. In some embodiments, the antibodies have *in vivo* therapeutic and/or prophylactic uses against cancer.

Non-limiting examples of therapeutic and prophylactic antibodies that can be used in combination with a benzo[b]azepine TLR agonist formulation of the invention include MDX-010 (Medarex, NJ) which is a humanized anti-CTLA-4 antibody currently in clinic for the treatment of prostate cancer; SYNAGIS® (MedImmune, MD) which is a humanized anti-respiratory syncytial virus (RSV) monoclonal antibody for the treatment of RSV infection; and HERCEPTIN® (Trastuzumab) (Genentech, CA) which is a humanized anti-HER2 monoclonal antibody for the treatment of metastatic breast cancer. Other examples are humanized anti-CD18 F(ab')₂ (Genentech); CDP860 which is a humanized anti-CD18 F(ab')₂ (Celltech, UK); PRO542 which is an anti-HIV gp120 antibody fused with CD4 (Progenics/Genzyme Transgenics); Ostavir which is a human anti-Hepatitis B virus antibody (Protein Design Lab/Novartis); PROTOVIR™ which is a humanized anti-CMV IgG1 antibody (Protein Design Lab/Novartis); MAK-195 (SEGARD) which is a murine anti-TNF-α F(ab')₂ (Knoll Pharma/BASF); IC14 which is an anti-CD 14 antibody (ICOS Pharm); a humanized anti-VEGF IgG1 antibody (Genentech); OVAREX™ which is a murine anti-CA 125 antibody (Altarex); PANOREX™ which is a murine anti-17-IA cell surface antigen IgG2a antibody (Glaxo Wellcome/Centocor); BEC2 which is a murine anti-idiotype (GD3 epitope) IgG antibody (ImClone System); IMC-C225 which is a chimeric anti-EGFR IgG antibody (ImClone System); VITAXIN™ which is a humanized anti-αVβ3 integrin antibody (Applied Molecular Evolution/MedImmune); Campath 1H/LDP-03 which is a humanized anti-CD52 IgG1 antibody (Leukosite); Smart M195 which is a humanized anti-CD33 IgG antibody (Protein Design Lab/Kanebo); RITUXAN™ which is a chimeric anti-CD20 IgG1 antibody (IDEC Pharm/Genentech, Roche/Zettyaku); LYMPHOCIDE™ which is a humanized anti-CD22 IgG antibody (Immunomedics); Smart ID10 which is a humanized anti-HLA antibody (Protein Design Lab); ONCOLYM™ (Lym-1) is a radiolabelled murine anti-HLA DIAGNOSTIC REAGENT antibody (Techniclone); ABX-IL8 is a human anti-IL8 antibody (Abgenix); anti-CD11a is a humanized IgG1 antibody (Genentech/Xoma); ICM3 is a humanized anti-ICAM3 antibody (ICOS Pharm); IDEC-114 is a primatized anti-CD80 antibody (IDEC Pharm/Mitsubishi); ZEVALIN™ is a radiolabelled murine anti-CD20 antibody (IDEC/Schering AG); IDEC-131 is a humanized anti-CD40L antibody (IDEC/Eisai); IDEC-151 is a primatized anti-CD4 antibody (IDEC); IDEC-152 is a primatized anti-CD23 antibody (IDEC/Seikagaku); SMART anti-CD3 is a humanized anti-CD3 IgG (Protein Design Lab); 5G1.1 is a humanized anti-complement factor 5 (C5) antibody (Alexion Pharm); D2E7 is a humanized anti-TNF-α antibody (CAT/BASF); CDP870 is a humanized anti-TNF-α Fab fragment (Celltech); IDEC-151 is a primatized anti-CD4 IgG1 antibody (IDEC Pharm/SmithKline Beecham); MDX-CD4 is a human anti-CD4 IgG antibody (Medarex/Eisai/Genmab); CDP571 is a humanized anti-TNF-α IgG4 antibody (Celltech); LDP-02 is a humanized anti-α4β7 antibody (LeukoSite/Genentech); OrthoClone OKT4A is a humanized anti-CD4 IgG antibody (Ortho Biotech); ANTOVA™ is a humanized anti-CD40L IgG antibody (Biogen); ANTEGREN™ is a humanized anti-VLA-4 IgG antibody (Elan); MDX-33 is a human anti-CD64 (FcγR) antibody (Medarex/Centeon); SCH55700 is a humanized anti-IL-5 IgG4 antibody (Celltech/Schering); SB-240563 and SB-240683 are humanized anti-IL-5 and IL-4 antibodies, respectively, (SmithKline Beecham); rhuMab-E25 is a humanized anti-IgE IgG1 antibody (Genentech/Norvartis/Tanox Biosystems); ABX-CBL is a murine anti CD-147 IgM antibody (Abgenix); BTI-322 is a rat anti-CD2 IgG antibody (Medimmune/Bio Transplant); Orthoclone/OKT3 is a murine anti-CD3 IgG2a antibody (ortho Biotech); SIMULECT™ is a chimeric anti-CD25 IgG1 antibody (Novartis Pharm); LDP-01 is a humanized anti-β₂-integrin IgG antibody (LeukoSite); Anti-LFA-1 is a murine anti CD18 F(ab')₂ (Pasteur-Merieux/Immunotech); CAT-152 is a human anti-TGF-β₂ antibody (Cambridge Ab Tech); and Corsevin M is a chimeric anti-Factor VII antibody (Centocor). The above-listed immunoreactive reagents, as well as any other immunoreactive reagents, may be administered according to any regimen known to those of skill in the art, including the regimens recommended by the suppliers of the immunoreactive reagents.

### 1.2.1.5 Combination with other therapeutic agents

In addition to anti-cancer agents and therapeutic antibodies, VTX-2337 of the invention can be administered in combination with other therapeutic agents such as anti-angiogenic agents (e.g., in methods for the treatment of solid tumors and for the treatment and prevention of metastases) and anti-hormonal agents (particularly in methods for the treatment of hormone-dependent cancers such as breast cancer and prostate cancer).

In one embodiment, VTX-2337 of the invention is administered in combination with one or more anti-angiogenic agents. Such agents include, without limitation, angiostatin, thalidomide, kringle 5, endostatin, Serpin (Serine Protease Inhibitor) anti-thrombin, 29 kDa N-terminal and a 40 kDa C-terminal proteolytic fragments of fibronectin, 16 kDa proteolytic fragment of prolactin, 7.8 kDa proteolytic fragment of platelet factor-4, a 13-amino acid peptide corresponding to a fragment of platelet factor-4 (Maione et al., 1990, Cancer Res. 51:2077-2083), a 14-amino acid peptide corresponding to a fragment of collagen I (Tolma et al., 1993, J. Cell Biol. 122:497-511), a 19 amino acid peptide corresponding to a fragment of Thrombospondin I (Tolsma et al., 1993, J. Cell Biol. 122:497-511), a 20-amino acid peptide corresponding to a fragment of SPARC (Sage et al., 1995, J. Cell. Biochem. 57:1329-1334), or any fragments, family members, or variants thereof, including pharmaceutically acceptable salts thereof.

Other peptides that inhibit angiogenesis and correspond to fragments of laminin, fibronectin, procollagen, and EGF have also been described (*see*, *e.g*., Cao, 1998, Prog Mol Subcell Biol. 20:161-176). Monoclonal antibodies and cyclic pentapeptides, which block certain integrins that bind RGD proteins (*i.e*., possess the peptide motif Arg-Gly-Asp), have been demonstrated to have anti-vascularization activities (Brooks et al., 1994, Science 264:569-571; Hammes et al., 1996, Nature Medicine 2:529-533). Moreover, inhibition of the urokinase plasminogen activator receptor by receptor antagonists inhibits angiogenesis, tumor growth and metastasis (Min et al., 1996, Cancer Res. 56: 2428-33; Crowley et al., 1993, Proc Natl Acad Sci. 90:5021-25).

In another embodiment, VTX-2337 of the invention is used in association with a hormonal treatment modality. Such treatment modalities include the administration of hormonal antagonists (*e.g*., flutamide, bicalutamide, tamoxifen, raloxifene, leuprolide acetate (LUPRON), LH-RH antagonists), inhibitors of hormone biosynthesis and processing, and steroids (*e.g*., dexamethasone, retinoids, deltoids, betamethasone, cortisol, cortisone, prednisone, dehydrotestosterone, glucocorticoids, mineralocorticoids, estrogen, testosterone, progestins), vitamin A derivatives (e.g., all-trans retinoic acid (ATRA)); vitamin D3 analogs; antigestagens (*e.g*., mifepristone, onapristone), and antiandrogens *(e.g*., cyproterone acetate).

In another embodiment, VTX-2337 of the invention is used in association with a treatment modality that utilizes polynucleotide compounds, such as antisense polynucleotides, ribozymes, RNA interference molecules, triple helix polynucleotides and the like.

### 1.2.1.6 Combination with immunoregulatory agents

In certain embodiments, VTX-2337 of the invention is administered in combination with an immunoregulatory agent. In some embodiments, the benzo[b]azepine TLR agonist is formulated with the immunoregulatory agent. An "immunoregulatory agent" is a substance that suppresses, masks, or enhances the immune system of the subject to whom it is administered. Exemplary agents are those that suppress cytokine production, downregulate or suppress self-antigen expression, or mask the MHC antigens. Examples of such agents include 2-amino-6-aryl-5-substitutedpyrimidines (*see*, U.S. Pat. No. 4,665,077), azathioprine (or cyclophosphamide, if there is an adverse reaction to azathioprine); bromocryptine; glutaraldehyde (which masks the MHC antigens, as described in U.S. Pat. No. 4,120,649); anti-idiotypic antibodies for MHC antigens and MHC fragments; cyclosporin A; steroids such as glucocorticosteroids, *e.g*., prednisone, methylprednisolone, and dexamethasone; cytokine or cytokine receptor antagonists including anti-interferon-γ, -β, or -α antibodies; anti-tumor necrosis factor-α antibodies; anti-tumor necrosis factor-β antibodies; anti-interleukin-2 antibodies and anti-IL-2 receptor antibodies; anti-L3T4 antibodies; heterologous anti-lymphocyte globulin; panT antibodies, preferably anti-CD3 or anti-CD4/CD4a antibodies; soluble peptide containing a LFA-3 binding domain; streptokinase; TGF-β; streptodornase; FK506; RS-61443; deoxyspergualin; and rapamycin. Examples of cytokines include, but are not limited to lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α; mullerian-inhibiting substance; mouse gonadotropin-associated peptide; inhibin; activin; vascular endothelial growth factor; integrin; thrombopoiotin (TPO); nerve growth factors such as NGF-α; platelet-growth factor; transforming growth factors (TGFs) such as TGF-α and TGF-α; insulin-like growth factor-I and -II; erythropoietin (EPO); osteoinductive factors; interferons; colony stimulating factors (CSFs) such as macrophage-CSF (M-CSF); granulocyte-macrophage-CgP (GM-CSP); and granulocyte-CSF (G-CSF); interleukins (ILs) such as IL-1, IL-la, IL-2, 1L-3, IL-4, IL-5, IL-6, IL-7, IL-8, IL-9, IL-1 I, IL-12, IL-15; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

In certain embodiments, the methods further include administering to the subject one or more immunomodulatory agents, preferably a cytokine. Preferred cytokines are selected from the group consisting of interleukin-1 (IL-1), IL-2, IL-3, IL-12, IL-15, IL-18, G-CSF, GM-CSF, thrombopoietin, and γ interferon.

### 1.2.1.7 Combination with compounds that enhance monocyte or macrophage function

In certain embodiments, a compound that enhances monocyte or macrophage function (*e.g*., at least about 25%, 50%, 75%, 85%, 90%, 9% or more) can be used in conjunction with the benzo[b]azepine TLR agonist formulations of the invention. Such compounds are known in the art and include, without limitation, cytokines such as interleukins (*e.g*., IL-12), and interferons (*e.g*., alpha or gamma interferon).

In certain embodiments, the compound that enhances monocyte or macrophage function is formulated with VTX-233 and is thus administered concurrently with VTX-2337.

In other embodiments, the compound that enhances monocyte or macrophage function is administered separately from VTX-2337 and can be administered concurrently (within a period of hours of each other), during the same course of therapy, or sequentially with VTX-2337. In such embodiments, the compound that enhances monocyte or macrophage function is preferably administered to a human subject. In one embodiment, the human subject has a blood leukocyte, monocyte, neutrophil, lymphocyte, and/or basophil count that is within the normal range for humans. Normal ranges for human blood leukocytes (total) is about 3.5-10.5 (10⁹/L). Normal ranges for human blood neutrophils is about 1.7-7.0 (10⁹/L), monocytes is about 0.3-0.9 (10⁹/L), lymphocytes is about 0.9-2.9 (10⁹/L), basophils is about 0-0.3 (10⁹/L), and eosinophils is about 0.05-0.5 (10⁹/L). In other embodiments, the human subject has a blood leukocyte count that is less than the normal range for humans, for example at least about 0.01, 0.05, 0.1, 0.2, 0.3, 0.4, 0.5, 0.6, 0.7, or 0.8 (10⁹/L) leukocytes.

### 1.2.2 Target Cancers

The type of cancer that is treated by the methods of the present invention is a solid cancer such as ovarian cancer, breast cancer, head and neck cancer, renal cancer, bladder cancer, hepatocellular cancer, colorectal cancer, or lymphoma, or any combination thereof. Other types of cancers that can be treated by the methods of the present invention include, but are not limited to human sarcomas and carcinomas, *e.g*., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, hepatoma, bile duct carcinoma, choriocarcinoma, seminoma, embryonal carcinoma, Wilms' tumor, cervical cancer, testicular tumor, lung carcinoma, small cell lung carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, retinoblastoma; leukemias, *e.g*., acute lymphocytic leukemia and acute myelocytic leukemia (myeloblastic, promyelocytic, myelomonocytic, monocytic and erythroleukemia); chronic leukemia (chronic myelocytic (granulocytic) leukemia and chronic lymphocytic leukemia); and polycythemia vera, lymphoma (Hodgkin's disease and non-Hodgkin's disease), multiple myeloma, Waldenström's macroglobulinemia, and heavy chain disease.

### 1.3 Administration and Dosing

VTX-2337 of the invention is preferably formulated for injection, most preferably subcutaneous administration. In certain embodiments, VTX-2337 of the invention is formulated for administration by an intradermal, a transdermal, an intravenous, or an intramuscular route.

The formulations of the present invention contain an amount of VTX-2337 that is effective for the intended use. Particular dosages are also selected based on a number of other factors including the age, sex, species and condition of the patient. Effective amounts can also be extrapolated from dose-response curves derived from in vitro test systems or from animal models.

In certain embodiments, the dose of VTX-2337 is measured in units of mg/kg of body weight. In other embodiments, the dose is measured in units of mg/kg of lean body weight (i.e., body weight minus body fat content). In other embodiments, the dose is measured in units of mg/m² of body surface area. In other embodiments, the dose is measured in units of mg per dose administered to a patient. Any measurement of dose can be used in conjunction with the compositions and methods of the invention and dosage units can be converted by means standard in the art.

Examples of dosing regimens that can be used in the methods of the invention include, but are not limited to, daily, three times weekly (intermittent), weekly, or every 14 days. In certain embodiments, dosing regimens include, but are not limited to, monthly dosing or dosing every 6-8 weeks. In a preferred embodiment, a benzo[b]azepine TLR agonist formulation of the present invention is administered by subcutaneous injection weekly or biweekly in combination with a suitable treatment modality for the treatment of cancer in a subject, preferably a human subject.

Exemplary doses of VTX-2337 include milligram amounts per kilogram of the subject. In one embodiment, the dose is from about 0.02 to 10 mg/kg of body weight or about 0.04 to 5 mg/kg of body weight. In a specific embodiment, the dosage is about 0.05 mg/kg, about 0.1 mg/kg, about 0.5 mg/kg, about 1 mg/kg, about 5 mg/kg, or about 10 mg/kg of the subject's body weight.

In certain embodiments of the methods for treating cancer, VTX-2337 is administered alone or in the combinational therapy for cancer treatment to the subject at a dose of from about 0.02 to 10 mg/kg of body weight or about 0.04 to 5 mg/kg of body weight of the subject. In particular embodiments, the benzo[b]azepine TLR agonist is administered at a dose of about 0.05 mg/kg, about 0.1 mg/kg, about 0.5 mg/kg, about 1 mg/kg, about 5 mg/kg, or about 10 mg/kg of the subject's body weight. In certain further embodiments, VTX-2337 is administered to the subject on a weekly or biweekly basis. In specific embodiments, a daily dose is at least 0.05 mg, 0.50 mg, 1.0 mg, 5.0 mg, 10 mg, 15 mg, 20 mg, 30 mg, or at least 50 mg.

In some embodiments, the dose for the benzo[b]azepine TLR8 agonist (e.g., VTX-2337) administered alone or in the combinational therapy for cancer treatment is between 0.1-10 mg/m² (*e.g*., 0.1-0.3 mg/m², 0.1-3.9 mg/m², 0.1-1 mg/m², 0.1-2 mg/m², 0.1-4 mg/m², 2-4 mg/m², 2.5-3.5 mg/m², 2-6 mg/m², 2-8 mg/m²). This includes 0.1 mg/m², 1 mg/m², 2 mg/m², 3 mg/m², 4 mg/m², 5 mg/m², 6 mg/m², 7 mg/m², 8 mg/m² and points in-between. It is noted that 2.5-3.5 mg/m² corresponds to ∼ 0.05-0.075 mg/kg if one assumes a body surface area of 1.5 m² corresponds to a body weight of 70 kg. The frequency of administration is preferably once every 7 to 21 days (*e.g*., once every 7, 10, 14, 18, 21 days). In some embodiments, the frequency of administration is preferably 1, 2, or 3 times every 7 to 21 days (*e.g*., once every 7, 10, 14, 18, 21 days). The benzo[b]azepine TLR agonist may be given until disease progression or unacceptable toxicity. In some embodiments, 2-20 doses are given (*e.g*., 2, 4, 6, 8, 10, 12, 14, 16, 18, 20 doses). The preferred route of administration is subcutaneous.

In certain embodiments of the methods for treating cancer, doxorubicin is administered alone or in the combinational therapy of the invention to the subject at a dose of from about 0.02 to 10 mg/kg of body weight or about 0.04 to 5 mg/kg of body weight of the subject or not more than 50 mg/m² of the body surface area of the subject.

Recommended dosages for intradermal, intramuscular, intraperitoneal, subcutaneous, epidural, or intravenous administration are in the range of about 0.02 to 10 mg/kg of body weight per day. Suitable doses for topical administration are in the range of about 0.001 milligram to about 50 milligrams, depending on the area of administration. Those skilled in the art will appreciate that dosages are generally higher and/or frequency of administration greater for initial treatment as compared with maintenance regimens.

Doxorubicin is preferably formulated for injection, most preferably intravenous administration. In certain embodiments, doxorubicin is formulated for administration by an intradermal, a transdermal, a subcutaneous, or an intramuscular route.

In certain embodiments, the dose of doxorubicin is measured in units of mg/kg of body weight. In other embodiments, the dose is measured in units of mg/kg of lean body weight (i.e., body weight minus body fat content). In other embodiments, the dose is measured in units of mg/m² of body surface area. In other embodiments, the dose is measured in units of mg per dose administered to a patient. Any measurement of dose can be used in conjunction with the compositions and methods of the invention and dosage units can be converted by means standard in the art.

In certain embodiments, doxorubicin is administrated prior to, concurrently with, or subsequent to the administration of VTX-2337.

In certain embodiments of the methods for treating cancer, doxorubicin is administered to the subject at a dose of from about 0.02 to 10 mg/kg of body weight or about 0.04 to 5 mg/kg of body weight of the subject.

### 1.3.1 Exemplary Regimens for the treatment of cancer

In particular embodiments, VTX-2337 formulations of the invention are used in combination with an existing treatment regimen for the treatment of cancer in a subject, preferably a human subject. In accordance with this embodiment, the benzo[b]azepine TLR agonist formulation can be administered prior to, subsequently, or concurrently with a suitable anti-cancer agent(s) for the treatment of cancer. Preferably, the administration of VTX-2337 is coordinated with the dosage and timing of the anti-cancer agent(s) depending on the type of cancer, the subject's history and condition, and the particular anti-cancer agent(s) of choice.

In one embodiment, the regimen comprises 5-fluorouracil, cisplatin, docetaxel, HERCEPTIN®, gemcitabine, IL-2, paclitaxel, and/or VP-16 (etoposide) for the treatment of breast cancer. In another embodiment, the regimen comprises paclitaxel, docetaxel, mitoxantrone, and/or an androgen receptor antagonist (*e.g*., flutamide) for the treatment of prostate cancer. In another embodiment, the regimen comprises fludarabine, cytosine arabinoside, gemtuzumab (MYLOTARG), daunorubicin, methotrexate, vincristine, 6-mercaptopurine, idarubicin, mitoxantrone, etoposide, asparaginase, prednisone and/or cyclophosphamide for the treatment of leukemia. In one embodiment, the regimen comprises dexamethasone for the treatment of myeloma. In one embodiment, the regimen comprises dacarbazine for the treatment of melanoma. In one embodiment, the regimen comprises irinotecan for the treatment of colorectal cancer. In one embodiment, the regimen comprises paclitaxel, docetaxel, etoposide and/or cisplatin for the treatment of lung cancer. In one embodiment, the regimen comprises cyclophosphamide, CHOP, etoposide, bleomycin, mitoxantrone and/or cisplatin for the treatment of non-Hodgkin's lymphoma. In one embodiment, the regimen comprises cisplatin for the treatment of gastric cancer. In one embodiment, the regimen comprises gemcitabine for the treatment of pancreatic cancer.

The duration of treatment with the anti-cancer agent may vary according to the particular therapeutic agent used. In certain embodiments, the administration is discontinuous, i.e., daily doses are divided into several partial administrations. According to certain embodiments, the method of treatment comprises at least one cycle, preferably more than one cycle, during which a single therapeutic or sequence of therapeutics is administered. An appropriate period of time for one cycle can be determined according to routine methods by the skilled artisan, as well as the total number of cycles, and the interval between cycles.

In a specific embodiment, the regimen comprises gemcitabine at a dose ranging from 100 to 1000 mg/m²/cycle. In another embodiment, the regimen comprises dacarbazine at a dose ranging from 200 to 4000 mg/m²/cycle. In a preferred embodiment, the dose of dacarbazine ranges from 700 to 1000 mg/m²/cycle. In another embodiment, the regimen comprises fludarabine at a dose ranging from 25 to 50 mg/m²/cycle. In another embodiment, the regimen comprises cytosine arabinoside (Ara-C) at a dose ranging from 200 to 2000 mg/m²/cycle. In another embodiment, the regimen comprises docetaxel at a dose ranging from 1.5 to 7.5 mg/kg/cycle. In another embodiment, the regimen comprises paclitaxel at a dose ranging from 5 to 15 mg/kg/cycle. In another embodiment, the regimen comprises cisplatin at a dose ranging from 5 to 20 mg/kg/cycle. In another embodiment, the regimen comprises 5-fluorouracil at a dose ranging from 5 to 20 mg/kg/cycle. In another embodiment, the regimen comprises doxorubicin at a dose ranging from 2 to 8 mg/kg/cycle. In another embodiment, the regimen comprises epipodophyllotoxin at a dose ranging from 40 to 160 mg/kg/cycle. In another embodiment, the regimen comprises cyclophosphamide at a dose ranging from 50 to 200 mg/kg/cycle. In another embodiment, the regimen comprises irinotecan at a dose ranging from 50 to 75, 75 to 100, 100 to 125, or 125 to 150 mg/m²/cycle. In another embodiment, the regimen comprises vinblastine at a dose ranging from 3.7 to 5.4, 5.5 to 7.4, 7.5 to 11, or 11 to 18.5 mg/m²/cycle. In another embodiment, the regimen comprises vincristine at a dose ranging from 0.7 to 1.4, or 1.5 to 2 mg/m²/cycle. In yet another embodiment, the regimen comprises methotrexate at a dose ranging from 3.3 to 5, 5 to 10, 10 to 100, or 100 to 1000 mg/m²/cycle.

In one embodiment, the regimen encompasses the use of a low dose of a chemotherapeutic agent. In accordance with this embodiment, initial treatment of a subject with VTX-2337 of the invention increases the sensitivity of a tumor to subsequent challenge with an anti-cancer agent. Thus, the anti-cancer agent can be administered to the subject at a dose that is near or below the lower range of acceptable dosages for that agent administered alone. In one embodiment, the regimen comprises the subsequent administration of docetaxel at 6 to 60 mg/m²/day or less. In another embodiment, the regimen comprises the subsequent administration of paclitaxel at 10 to 135 mg/m²/day or less. In another embodiment, the regimen comprises the subsequent administration of fludarabine at 2.5 to 25 mg/m²/day or less. In another embodiment, the regimen comprises the subsequent administration of cytosine arabinoside (Ara-C) at 0.5 to 1.5 g/m²/day or less. In another embodiment, the regimen comprises the subsequent administration of gemcitabine at from 10 to 100mg/m²/cycle. In another embodiment, the regimen comprises the subsequent administration of cisplatin, *e.g*., PLATINOL or PLATINOL-AQ (Bristol Myers), at a dose ranging from 5 to 10, 10 to 20, 20 to 40, or 40 to 75 mg/m²/cycle. In another embodiment, the regimen comprises the subsequent administration of cisplatin ranging from 7.5 to 75 mg/m²/cycle. In another embodiment, the regimen comprises the subsequent administration of carboplatin, *e.g*., PARAPLATIN (Bristol Myers), at a dose ranging from 2 to 4, 4 to 8, 8 to 16, 16 to 35, or 35 to 75 mg/m²/cycle. In another embodiment, the regimen comprises the subsequent administration of docetaxel, *e.g*., TAXOTERE (Rhone Poulenc Rorer) at a dose ranging from 6 to 10, 10 to 30, or 30 to 60 mg/m²/cycle. In another embodiment, the regimen comprises the subsequent administration of paclitaxel, *e.g*., TAXOL (Bristol Myers Squibb), at a dose ranging from 10 to 20, 20 to 40, 40 to 70, or 70 to 135 mg/kg/cycle. In another embodiment, the regimen comprises the subsequent administration of 5-fluorouracil at a dose ranging from 0.5 to 5 mg/kg/cycle. In another embodiment, the regimen comprises the subsequent administration of doxorubicin, *e.g*., ADRIAMYCIN (Pharmacia & Upjohn), DOXIL (Alza), RUBEX (Bristol Myers Squibb), at a dose ranging from 2 to 4, 4 to 8, 8 to 15, 15 to 30, or 30 to 60 mg/kg/cycle.

The above-described administration schedules are provided for illustrative purposes only and should not be considered limiting.

### 1.4 Kits

The present invention provides a pharmaceutical pack or kit comprising one or more containers filled with a liquid or lyophilized VTX-2337 and/or doxorubicin. In preferred embodiments the liquid or lyophilized formulation is sterile. In one embodiment, the kit comprises a liquid or lyophilized formulation of the invention, in one or more containers, and one or more other prophylactic or therapeutic agents useful for the treatment of cancer or an infectious disease. The one or more other prophylactic or therapeutic agents may be in the same container as VTX-2337 or in one or more other containers. Preferably, VTX-2337 is formulated at a concentration of from about 0.5 mg/ml to about 50 mg/ml, from about 1 mg/ml to about 40 mg/ml, or from about 2 mg/ml to about 15 mg/ml, and the formulation is suitable for injection, preferably subcutaneous injection. Preferably, the kit contains VTX-2337 in unit dosage form. Most preferably, the unit dosage form is in a form suitable to provide a unit dose of about 0.02 to 10 mg/kg or about 0.04 to 5 mg/kg of body weight of the subject to be treated.

In certain embodiments, the kit further comprises instructions for use in the treatment of cancer (*e.g*., using the liquid formulations of the invention alone or in combination with another prophylactic or therapeutic agent), as well as side effects and dosage information for one or more routes of administration. Optionally associated with such container(s) is a notice in the form prescribed by a governmental agency regulating the manufacture, use or sale of pharmaceuticals or biological products, which notice reflects approval by the agency of manufacture, use or sale for human administration.

All publications and patent documents cited herein are incorporated herein by reference as if each such publication or document was specifically and individually indicated to be incorporated herein by reference. Citation of publications and patent documents is not intended as an admission that any is pertinent prior art, nor does it constitute any admission as to the contents or date of the same.

The invention is further defined by reference to the following examples, which are not meant to limit the scope of the present invention. It will be apparent to those skilled in the art that many modifications, both to the materials and methods, may be practiced without departing from the purpose and interest of the invention.

### 1.5 Examples

### Example 1: TLR8 Agonist and Doxil chemotherapy potently activate human antitumor immune response in a human immune system mouse model

Because of differences between mouse and human immune systems, many of the effects of immunomodulatory drugs cannot be fully studied in syngeneic mouse models. A novel tumor-bearing mouse model with human immune system (HIS) was generated to study interactions between chemotherapy and immune modulatory therapy. The individual effects and the interactions between doxorubicin, a drug which induces immunogenic tumor cell death and activates antigen-presenting cells, and VTX-2337, a TLR8 agonist, which induces potent activation and type 1 polarization of human myeloid DCs were tested and showed reduced activity on murine leukocytes. Nod/SCID/ILRγc knock out (NSG) mice were inoculated with human CD34+ cord blood cells from HLA-A2+ human donors; transplanted s.c. with human HLA-A2+ OVCAR5 ovarian cancer tumors; and treated with pegylated liposomal doxorubicin (Doxil or PLD); VTX-2337; or the two agents in combination. NSG-HIS mice exhibited a full human hematopoietic system, including human monocytes, macrophages and plasmacytoid and myeloid DCs as well as T cell subsets. In NSG-HIS mice, VTX-2337 induced dose-dependent activation of human CD14+ and CD11c+ cells in vivo within 6 hrs. Transient, dose-dependent upregulation of human Th1 cytokines but also IL-10 was observed in the plasma of mice treated with VTX-2337, reaching peaks within 6 hrs and subsiding within 24 hrs. Doxil alone also induced mild activation of CD11c+ DCs in vivo and mild upregulation of Th1 cytokines. The combination of two drugs induced potent activation of CD11c+ DCs and monocytes, and markedly increased Th1 cytokines but not IL-10. HLA-A2+ OVCAR5 tumors were successfully engrafted, exhibiting infiltration by human leukocytes. VTX-2337 and Doxil treatment independently induced tumor-infiltrating human leukocytes and restricted growth of human ovarian tumor xenografts in a dose-dependent manner, while the combination of the two drugs induced the highest frequency of tumor-infiltrating human leukocytes and potently restricted growth of ovarian tumors. Combined activation of innate and adaptive immunity by VTX-2337 and Doxil, as well as sensitization of tumor cells by Doxil to adaptive and innate immune effector mechanisms was at the basis of the observed interactions suppressing tumor growth. The NSG-HIS provided a suitable tool to establish interactions during TLR8 agonist and Doxil chemotherapy, and the results warrant clinical testing.

### Materials and Methods

### Reagents:

VTX-2337 formulation: 40 mg/mL of 2-amino-N,N-dipropyl-8-(4-(pyrrolidine-1-carbonyl)phenyl)-3H-benzo[b]azepine-4-carboxamide, which is formulated as an inclusion complex with 15% w/v Captisol^{®} (sulfobutyl ether β-cyclodextrin) in 10 mM citrate buffer (pH=6.5). The formulation was further diluted with 0.9% sterile sodium chloride to the appropriate concentrations prior to use.

PLD (i.e., Doxil, manufactured by Ben Venue Laboratories Inc Bedford OH 4414146) was purchased from the university of Pennsylvania hospital pharmacy.

**Generation of NSG-HIS mice:** All the in vivo mouse studies were approved by the University of Pennsylvania Institutional Animal Care and Use Committee according to National Institutes of Health (NIH) guidelines. NOD*-scid IL2rγ^{null}* (NSG) mice obtained from the University of Pennsylvania xenograft facility were previously irradiated (250 Rads), followed the next day by intravenous (i.v.) injection of T cell-depleted human cord blood cells containing 1-2×10⁵ CD34⁺ (LONZA, 2C-101). After approximately 3 months, levels of engraftment and reconstitution of the human hematopoietic system were verified by bleeding and hCD45 staining (BD Pharmingen, clone 2D1 cat# 557833 APC-CY7).

**Measurement of cytokines:** In some experiments NSG-HIS were injected subcutaneously (s.c.) with 0.5 or 5 mg/kg VTX-2337 alone or in combination with intraperitoneal (i.p.), PLD at the maximum tolerated dose (MTD, 50mg/m²). In other experiments, human PBMC were stimulated in vitro with VTX-2337. In all experiments, plasma or media supernatants were collected 6 hours after the VTX-2337 administration. Levels of cytokines induced by VTX-2337 or VTX-2337 plus PLD administration were measured both in vitro and in vivo by Rules Based Medicine (Austin, TX) using a Luminex-based technology that assesses the levels of 96 human analytes in either culture supernatants or plasma samples collected from treated animals.

**Treatment of OVCAR5 tumor bearing NSG-HIS mice:** OVCAR5 cells were injected s.c. (5x10⁶ cells) into HLA-A2⁺ CD34⁺ engrafted NSG-HIS mice. In untreated control mice, tumors progressively developed resulting in animal death within 90 days of tumor challenge. Tumors were measured two times per week, and the volume was calculated as follows: (length x length) x (width)/2. Tumor bearing mice were randomized into four treatment groups (n=8-10/group) when mean tumor volumes reached approximately 50 mm³, or ∼30 days after tumor cell implantation. Treatment groups consisted of a vehicle control, PLD (50 mg/m² i.p. given every two weeks), VTX-2337 (0.5 mg/kg s.c. given every other day three times for each cycle), or the combination of PLD and VTX-2337, with VTX-2337 treatment starting 5 days after PLD. Treatment cycles were 14 days in duration and three treatment cycles were administered to each group. Over the course of each treatment cycle, groups given PLD received the chemotherapy drug on Day 1, while groups receiving VTX-2337 alone or in combination with PLD received VTX-2337 on Days 5, 7, and 9 of cycle.

**Flow cytometry:** For flow cytometry analysis of leukocytes, tumors, bone marrow, or spleen were minced placed in 6 cm Petri dishes, transferred in 15 ml tubes, and incubated for 2h in a solution containing 2 mg/ml collagenase (Sigma #C9407) and DNAse (Sigma #D5025-15KO) in RPMI (Cellgro #1640CV) under continuous rotation. The suspension was passed through a 70-µm cell strainer using a syringe plunger, washed, centrifuged, and the pellet resuspended in PBS, 2% FBS (GIBCO # 10437). Following dissociation, 3-5x10⁶ cells were stained with 0.5 µg/ml of Ab for 30 min. at 4°C, washed, and analyzed by flow cytometry FACS-Canto (BD Pharmingen). Cells were stained using human hCD45 (BD Pharmingen clone 2D1 catalog# 557833 APC-CY7), hCD3 (Biolegend clone UCHT1 #300429 PerCP/Cy5.5), hCD4 (BD Pharmingen clone RPA-T4 #555349 APC), hCD8 (eBioscience clone RPA-T8 #11-0088 FITC), hCD11b (BD Pharmingen clone ICRF44 #555388 PE), hCD11c (Biolegend, clone 3.9 #301608 Pe-Cy7), hCD123 (BD Pharmingen clone 7G3 #558714 PerCP-Cy5.5), hCD14 (eBioscience clone 61D3 #25-0149 PE-Cy7), hCD40 (eBioscience clone 5C3 #11-0409 FITC) hCD80 (Biolegend clone 2D10 #305216 AF647) and hCD86 [BD Pharmingen clone 2331(FUN-1) #555658 PE].

**T cell expansion in vitro, reactivity selection and adoptive transfer study:** Tumor infiltrating leukocytes (TILs) were initially expanded from fragments of tumors from different treatment groups placed in culture with a high concentration of recombinant human interleukin 2 (rhIL-2, 600 IU/ml) as reported elsewhere (see, e.g., Dudley, M.E., et al. 2003. J Immunother 26:332-342 and Riddell, S.R., and Greenberg, P.D. 1990. J Immunol Methods 128:189-201). Briefly, fragments of tumors (∼2x2 mm) from different treatment groups were placed in AIMV media (GIBCO#12055) supplemented with 5% human serum (Valley Biomedical Inc #1017) and 600 I.U./ml hIL-2 (PeproTech #AF-200-02). Half of the media was replaced every 3 days until exponential growth was achieved; then the cultures were split as needed to keep the cell concentration within a range of ∼5x10⁵-1x10⁶ cells per mL. Once a sufficient number of cells were obtained, all the cultures were assessed for OVCAR5 reactivity in vitro. OVCAR5-specific reactive TILs were then expanded using techniques that have been described previously (see, e.g., Dudley, M.E., et al. 2003. J Immunother 26:332-342). Briefly, 2 × 10⁸ allogeneic, irradiated feeder cells (HLA-A2⁺ human PBMC) were combined with 30 µg/mL OKT3 antibody (eBioscience clone OKT3 #16-0037-85), 600 IU/mL rhIL-2 (PeproTech #AF-200-02), and 1 × 10⁶ TIL, mixed, and aliquoted into 175 cm² tissue culture flasks. Flasks were then incubated upright at 37 °C in 5% CO₂. On day 5, half the media was replaced with a 1:1 mixture of AIM V containing 600 IU/mL rhIL-2. Media was added to these flasks as needed to maintain the cell density at around 0.5-1 × 10⁶ cells/mL. Each initial well was considered to be an independent TIL culture and maintained separately from the others. In the adoptive transfer study, non-human CD34⁺ engrafted NSG mice were challenged s.c. with 5 × 10⁶ OVCAR5 cells 30 to 40 days after intravenous (i.v.) injection of 1x10⁷ expanded T cells.

**Cytokine Release and Cytotoxic Assays:** TIL activity and specificity were determined by analysis of cytokine secretion and direct CTL. For the interferon-γ (IFNγ) assay, TIL and control T-cell lines were washed twice prior to the coculture assay to remove rhIL-2. 1 × 10⁵ TILs and 1 × 10⁵ stimulator cells were plated in each well of a 96-well flat-bottom plate. TIL cultures were generally stimulated with OVCAR5 and two control HLA-A2⁺ melanoma tumor cell lines (526mel and 624mel). In some wells, to ensure MHC dependent activity, target cells were previously treated with anti-HLA A, B, and C neutralizing antibody (eBioscience clone w6/32 #16-9983-85). After overnight coculture, supernatants were harvested and IFNγ secretion was quantified by ELISA (Biolegend #430102). In the CTL assay, OVCAR5 were pulsed with chromium-55, and different ratios of TIL:target were plated in 96 well plates and incubated for 4 h. In some wells, OVCAR5 were previously incubated with anti HLA-A, B, and C neutralizing antibody. After incubation, 30 µl of media from the cocultures were spotted on Lumaplate and left to dry overnight. Radioactivity was detected by liquid scintillation counter Wallac 1450 Microbeta Plus.

**Cell viability Annexin V/7AAD:** To detect apoptosis, tumor cells were stained with annexin V/7AAD (BD Pharmingen #559763). Apoptotic cells were analyzed by flow cytometry according to the manufacturer's protocol. Briefly, OVCAR5 cells grown and subjected to different treatments were collected and pelleted at 1200 rpm and then washed twice with ice-cold PBS and re-suspended in a binding buffer (Pharmingen #51-66121 E) at a concentration of 1 × 10⁶ cells/mL; 100 µL of the solution (1 × 10⁵ cells) was transferred to each of two 5-mL culture tubes. Five µL of annexin V-PE (Pharmingen #51-65875Y) and added to each 100 µL solution, gently vortexed, incubated for 15 min at room temperature in the dark, washed with PBS, incubated with 5 µL of 7AAD (BD Pharmingen #51-68981E) for 10 minutes, and analyzed by FACS within 1 h.

**Protein extraction and western blotting analysis:** Total cellular protein was extracted on ice for 30 min in a lysis buffer [M-PER Mammalian Protein Extraction Reagent #78501 ThermoScientific]. 50 µg of protein from each sample was then denatured in 2X loading buffer at 100 °C for 5 min, separated on a sodium dodecyl sulfate polyacrylamide gel electrophoresis (SDS-PAGE) gel, and transferred onto a nitrocellulose membrane. The membranes were then incubated in 5% skim milk for 2 h at room temperature and then incubated with the first antibody

(Cell Signaling #3210 rabbit) overnight at 4 °C. The membranes were then washed with phosphate buffered saline (PBS) 0.5% Tween 20 (Sigma) three times and incubated with the secondary antibody (BioRad 172-1019) for 2 h at room temperature. Protein bands were visualized using ECL (Amersham #RPN2132) with X-films (Bioexpress # F-9023).

### TLR8 agonist activating anti-tumor effector mechanisms in human PBMC in vitro

VX-2337 is a selective and potent TLR8 agonist that effectively activates both human mDC and monocytes. Its activity is mainly restricted to these populations, and other human leukocyte populations are not activated directly, although indirect activation may ensue monocyte and DC activation. To test the global effects of TLR8 activation on human leukocytes, peripheral blood mononuclear cells (PBMC) from normal human volunteers (n=6) were incubated with VTX-2337 over a broad concentration range for 24 hours. High levels of TNFα, IFNγ and IL-12p70 were induced in response to TLR8 activation by VTX-2337, in a dose-dependent manner. Thus, a unique feature of TLR8 activation of PBMC is the induction of effector mediators that are known to play a critical role in cell mediated immune response to cancer. As a result, TLR8 agonists are useful for human immunotherapy.

### TLR8 agonist potently activating human APCs and drives Th1 immune activation in vivo in NSG-HIS mice

The activity of VTX-2337 was assessed in a novel murine model where NSG mice are reconstituted with human CD34+ cord blood cells. Once human hematopoietic stem cells reconstitute the bone marrow and begin hematopoiesis, the animals show within three to four months a complete reconstitution of human immune system, including B cells, CD3, CD4, CD8, NK, mDC, pDC and monocytes. Because of the immune-stimulatory effects seen in human PBMCs by VTX-2337 stimulation, the NSG-HIS mice were expected to be highly responsive to VTX-2337. Indeed, the administration of VTX-2337 to these mice replicated the immune-stimulatory effects already demonstrated in human PBMC with a dose-dependent increase in surface expression of multiple co-stimulatory molecules, including CD83, CD86 and MHC class II on engrafted human CD14+ monocytes CD11c, mDCs and CD123 pDCs. It was also demonstrated increased blood levels of human cytokines, known to be important in the immune response to tumors and already associated with TLR8 activation. These included IFNγ, TNFα and IL-12p40.

The structure of the TLR8 protein varies across species, and while VTX-2337 has activity in mice, the molecule was optimized for potency and selectively against human TLR8.

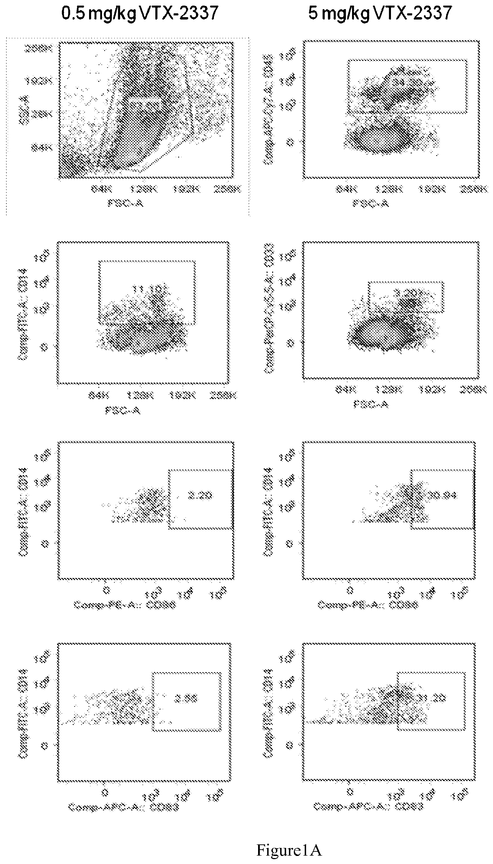

Because of the selectivity of VTX-2337 for human TLR8, NSG-HIS was chosen as a model of mouse host reconstituted with human hematopoietic system, to study the effects of VTX-2337 on human leukocytes in vivo. A high level of human hematolymphoid cell engraftment was seen in NSG-HIS mice 14 to 22 weeks following transfer of human cord blood CD34⁺ cells administered by the IV route to NSG mice at 6 weeks of age, as assessed by human (h)CD45 quantification in various compartments (Figure 1A); hCD45⁺ cells represented 35-75% of total cells in the blood, 40-68% of total cells in the spleen, and 40-70% of total cells in the bone marrow.

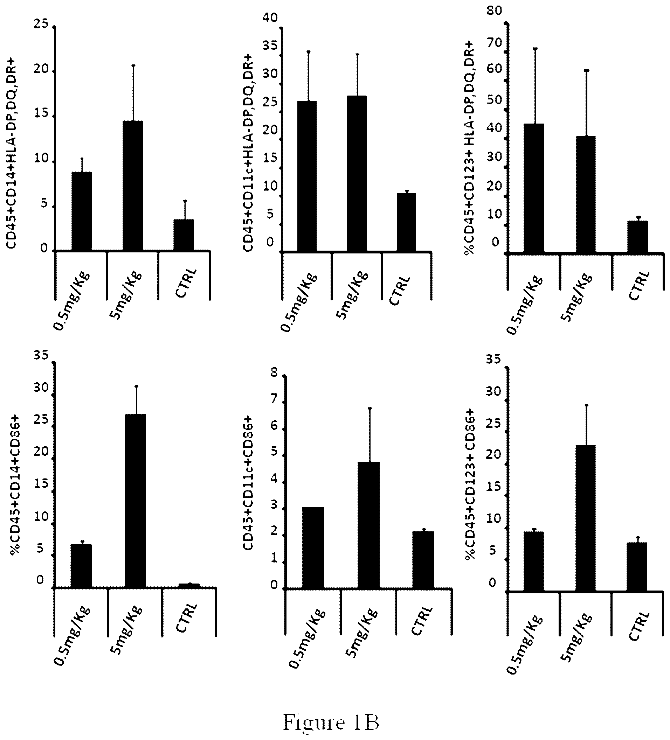

To demonstrate the activity of VTX-2337 in vivo, VTX-2337 was administered at 0.5 or 5 mg/kg s.c. to fully reconstituted NSG-HIS mice weeks post transplant. Spleen cells were collected six hours later to assess the levels of activation markers. Mice treated with either 0.5 or 5 mg/kg VTX-2337 demonstrated a marked increase in CD83, CD86 as well as MHC class II expression on monocytes (CD45⁺CD14⁺), myeloid DC (mDC, CD45⁺CD11c⁺) and plasmacytoid DC (pDC, CD45⁺CD123⁺) in comparison to control untreated mice (Figure 1B).

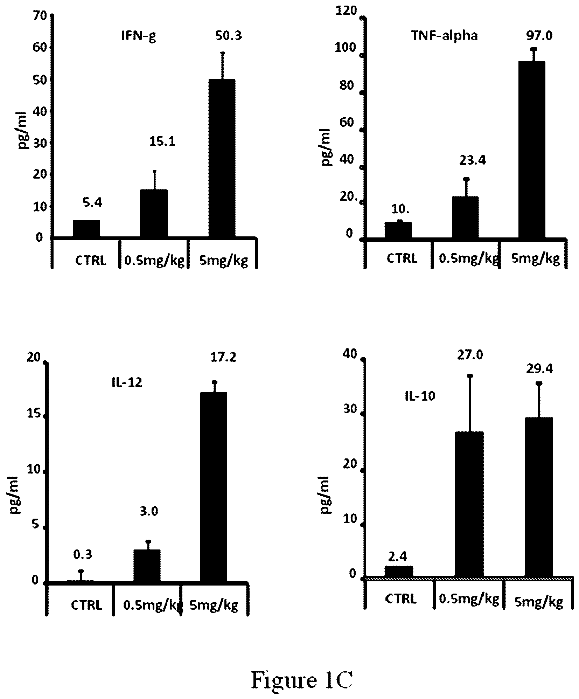

The human cytokine profile was examined in mouse plasma following a single s.c. administration of VTX-2337 (0.5 or 5 mg/kg) using a Luminex based assay (Figure 1C). A dode-dependent increase in the concentration of Th1 polarizing cytokines including IFNγ, TNFα and IL-12p40 was seen at six hours. Significant increase in IL-10 plasma levels was also detected in VTX-2337 treated animals in comparison to controls. Thus, VTX-2337 induced direct activation of the human monocyte/DC compartment and a subsequent potent Th1 activation of the human immune system in vivo, although increased IL-10 production was also seen.

### TLR8 activation following PLD resulting in Th1 cytokine response

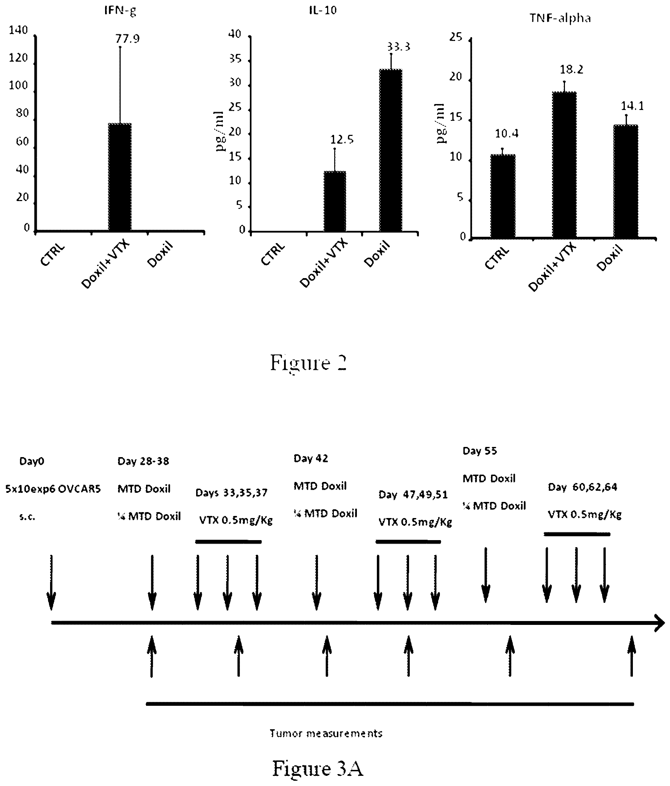

A "phased" administration schedule was tested, whereby PLD at maximally tolerated dose (MTD, 50 mg/m², i.p.) was administered first to 20-28-week old hCD34+ engrafted NSG mice, to inflict tumor cell damage and immunogenic antigen release; VTX-2337 (0.5 mg/kg) was administered five days later, to activate APCs. Levels of multiple cytokines were measured in the plasma 6 hours after VTX-2337 injection. Untreated NSG-HIS exhibited no detectable interferon gamma (IFNγ) in plasma. Similarly, treatment with PLD alone did not induce any IFNγ; however, the combination of VTX-2337 and PLD (Figure 2) induced significant levels of IFNγ, which were similar to those induced by VTX-2337 alone (Figure 1C). Furthermore, combination treatment with VTX-2337 and PLD induced TNFα upregulation over untreated controls, which was similar to either PLD or VTX-2337 alone. Thus, administration after PLD allowed VTX-2337 to induce a Th1 response. Importantly, although PLD as well as VTX-2337 induced IL-10 up-regulation when given alone (Figure 2), this effect was attenuated combining the two drugs (Figure 2). Overall, these data indicate that a 5-day interval between administration of PLD and VTX-2337 maintained the Th1 cytokine profile induced by VTX-2337 and in addition, reduced IL-10 levels. Thus, this combination induced an optimal cytokine response. The simultaneous administration of PLD and VTX-2337 produced negative interactions, as it suppressed IFNγ response.

An increase in plasma levels of TNFα and IL-10 demonstrated that the treatment of NSG-HIS mice with PLD induce immune activation. This immune activation is consistent with doxorubicin inducing "antigenic cell death" in both normal and tumor cells, and supports that TLR8 activation by VTX-2337 enhances the anti-tumor response. The administration of VTX-2337 and PLD^{®} resulted in both a reduction in plasma levels of the anti-inflammatory mediator IL-10 and increased IFNγ and TNFα levels.

### TLR8 activation enhancing the antitumor activity of PLD in vivo

This combination of MTD PLD and VTX-2337 at 0.5 mg/kg, a dose in mice comparable to what is being evaluated in clinical oncology studies, was subsequently used to treated tumor bearing NSG-HIS mice. The treatment regimen was designed to take advantage of the pharmacodynamic activities of PLD and VTX-2337, using multiple 14-day treatment cycles. Tumor-bearing NSG-HIS mice were initially given PLD to induce tumor cell death. This was followed 5 days later with multiple VTX-2337 treatments to activate immune scavenger cells, including mDC, monocytes, and macrophages that were removing dying tumor cells. As expected, PLD at the MTD resulted in a significant reduction in tumor growth rate relative to the vehicle control, while a small effect in tumor growth rate was seen with VTX 2337 alone. The combination of the two agents produced a marked decrease in the tumor growth rate over PLD alone over three 14-day treatment cycles.

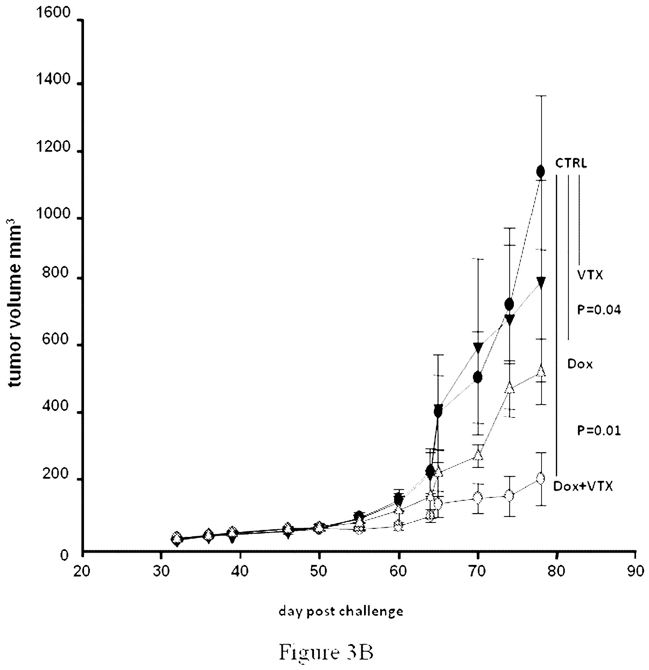

In the course of studying the effect of combining PLD with VTX-2337 on human ovarian cancer, hCD34 engrafted NSG-HIS-A2 mice were inoculated with HLA-A2⁺ matched human Ovarian Cancer Cell Line OVCAR5 (5x10⁶). Once tumors were well established, groups of mice (n=8-9/group) were treated with either vehicle, Doxil alone, VTX-2337 alone or the combination of VTX-2337 and Doxil. Interestingly, in spite of the immunomodulatory effects of VTX-2337, mice treated with the TLR8 agonist alone exhibited similar tumor growth as control untreated mice. As expected, mice treated with PLD at MTD (50 mg/m², i.p.) showed reduced tumor growth relative to control untreated mice. Importantly, there was a strong positive interaction between the two drugs; the effect of PLD was significantly enhanced by the combination with VTX-2337 (P=0.04) (Figure 3B), which almost completely suppressed tumor growth.

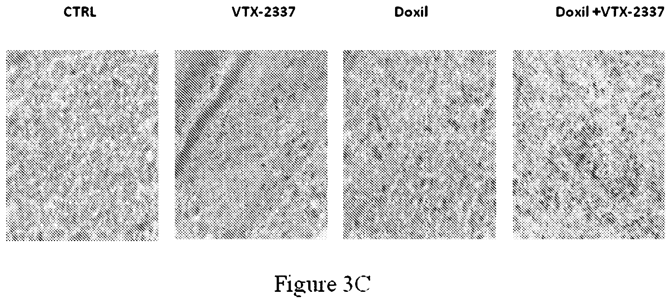

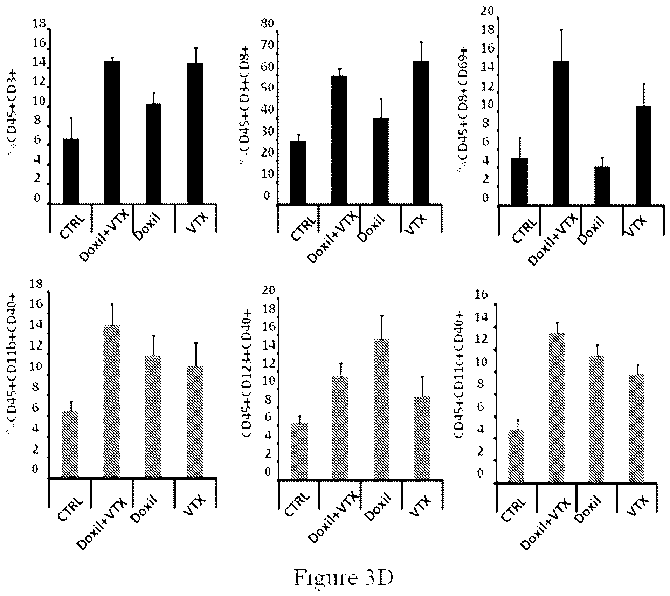

During the step of further characterizing this drug interaction, tumors were collected from each treatment group at the end of the study and were evaluated for leukocyte infiltration by immunohistochemistry and flow cytometry. Relatively few human CD45⁺ cells were present in tumors from the control group (Figure 3C), while all treatments resulted in increased CD45⁺ infiltration. Tumors from mice treated with the combination of PLD and VTX-2337 showed the greatest increase in infiltrating human CD45⁺ cells relative to either PLD or VTX-2337 alone (Figure 3C). Flow cytometry was used to further characterize the composition and maturation status of human leukocyte populations infiltrating tumors in the different groups. PLD alone did not induce significant changes in tumor-infiltrating lymphocytes over baseline. Interestingly, the TLR8 agonist induced a significant increase in total CD3⁺ T cells, in the percent of CD8⁺ T cells, as well as in the percent of CD69⁺ (activated) CD3⁺CD8⁺ T cells. The combination of PLD and TLR8 agonist induced similar changes. In addition, an increase was found in tumor-infiltrating CD40⁺ (activated) macrophages (CD45⁺CD11b⁺), pDC (CD45⁺CD123⁺), and mDC (CD45⁺CD11c⁺) in mice treated with VTX-2337, PLD alone or their combination (Figure 3D). Interestingly, there was a relative increase in the tumor-infiltrating macrophages to pDC ratio and mDC to pDC ratio in mice treated with the combination relative to each drug alone.

### TLR8 activation promoting the development of tumor-specific CTLs following PLD

The above results indicate a strong positive interaction between PLD and VTX-2337 against tumors. CD8⁺ T cell mediated rejection is a critical component of antitumor immune response and could be one of the mechanisms mediating the above interaction. Importantly, the VTX-2337 plus PLD combination produced effective tumor suppression.

During the course of investigating this interaction, the quality of the T cell infiltrate was analyzed in response to PLD, VTX-2337 or their combination. TILs from tumors collected from NSG-HIS-A2 mice treated with either drug alone, combination of PLD and VTX-2337 or control/vehicle were isolated and expanded using rhIL-2 (600 IU/mL). T cells were isolated from spleens of non-tumor bearing NSG-HIS-A2 mice as controls.

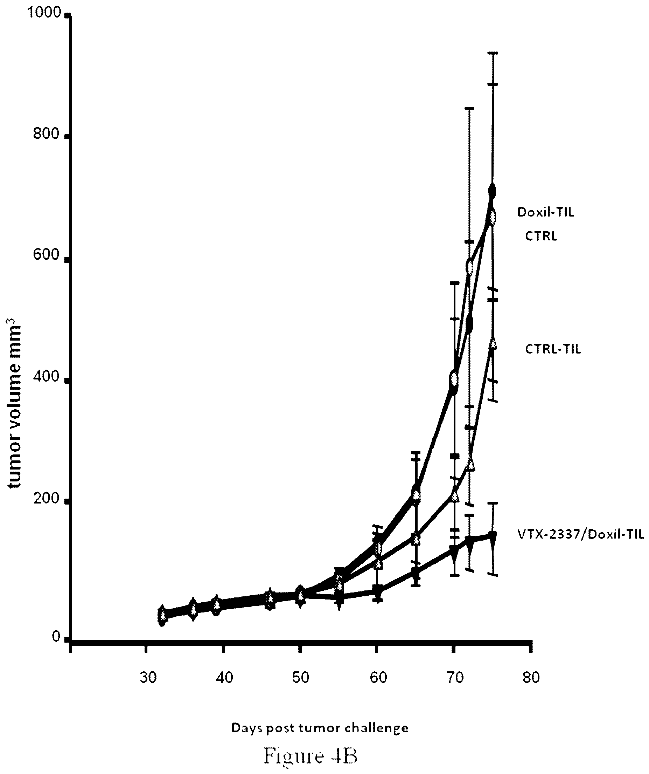

Ex vivo expanded TIL were transferred adoptively (on days 30 and 40 after tumor inoculation) into NSG mice bearing OVCAR5 tumors. TILs isolated from either the vehicle controls or PLD-treated donors, as well as T cells from spleens of non-tumor bearing mice, adoptively transferred into tumor-bearing recipients, failed to control tumor growth in recipient mice (Figure 4B). However, TILs from mice treated with the PLD and VTX-2337 combination ("PLD/VTX-2337" or "VTX-2337/Doxil") were able to effectively control the growth of OVCAR5 tumors in recipient mice (Figure 4B). These results confirm that in vivo, PLD and TLR8 agonist in combination elicit an effective T cell anti-tumor immune response.

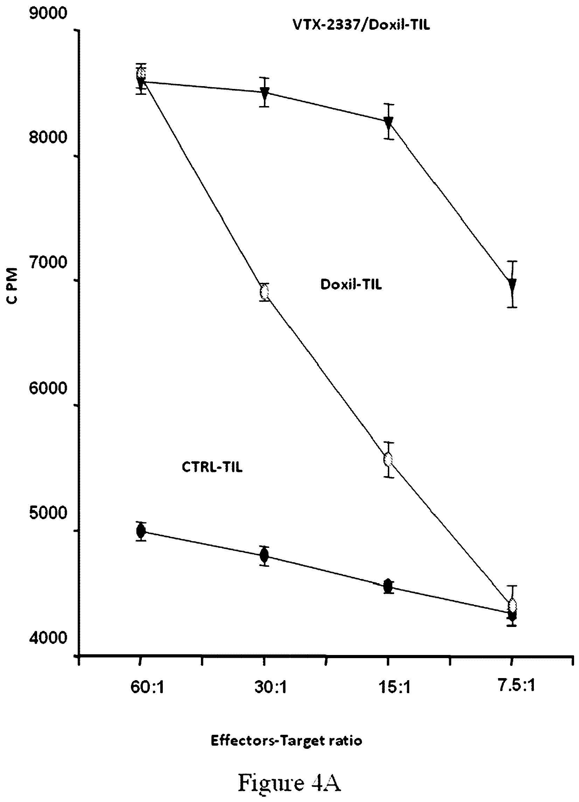

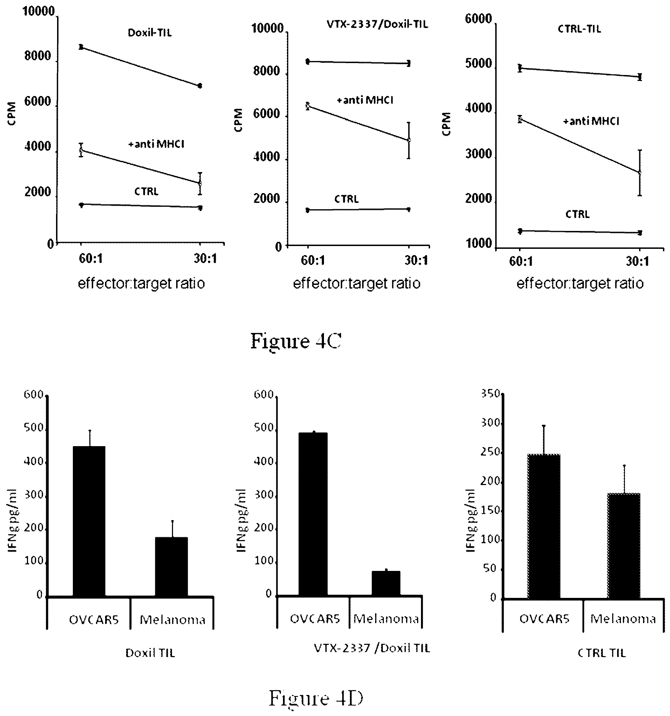

TILs were tested for the presence of tumor specific CTL in vitro. TIL from donor mice treated with the combination of PLD and VTX-2337 effectively lysed ⁵¹Cr labeled OVCAR5 target cells (Figure 4A), while TILs from donor mice treated with PLD alone had less lytic activity. Their capacity to lyse target cells was considerably greater than TILs from the control/vehicle treated group. In all treatment groups, target cell lysis by TIL was attributed to CTL responding to MHC-I restricted antigens, as the addition of anti-MHC class I neutralizing antibody reduced killing by the CTL (Figure 4C). TIL were co-cultured with either OVCAR5 cells or a melanoma cell line. TILs from both PLD and PLD/VTX-2337 treated donors released considerably more IFNγ in response to OVCAR5 cells than to melanoma cells (Figure 4D). TIL from control untreated mice produced minimal specific IFNγ in response to OVCAR5 stimulation. Lymphocytes expanded from the spleens of non-tumor bearing mice did not show cytolytic activity or IFNγ production in response to OVCAR5 cells.

CTLs (cytotoxic T cells or cytotoxic lymphocytes) from PLD/VTX-2337-treated mice had a much higher level of cytotoxic activity that those from mice treated with PLD alone, confirming that TLR8 activation of APCs enhances the development of anti-tumor specific T cells. The CTL activity was both MHC class I restricted and specific for OVCAR5, as demonstrated by the addition of mAb to MHC class I and the lack of activity towards irrelevant HLA-A2⁺ target cells. Using adoptive transfer experiments, it was demonstrated that the increase in tumor specific CTL activity resulting from the PLD/VTX-2337 treatment conferred anti-tumor activity in vivo. In non-reconstituted NSG tumor bearing mice adoptively transferred with 1x10⁷ T cells a day 30 and 40 days after challenge with OVCAR5, expanded tumor infiltrating cells from mice administered VTX-2337/PLD^{®}were able to effectively control tumor growth. Interestingly, cells derived from the tumors of mice treated with PLD^{®} alone were no more effective than cells from control treated mice.

Results further demonstrate that APC activation by VTX-2337 enhances the development of adaptive immune responses induced by anthracyclines. While the development of tumor-specific CTL is an important component of the therapeutic effect of VTX-2337 when given with PLD, the release of mediators with anti-tumor activity can be complementary. The release of high levels of IFNγ can activate NK cells, increasing lysis of tumor cells. The release of IL-12 by VTX-2337 also has important implications in the development of a successful immune response to tumors. This mediator is reported to activate NK cells, potentiate anti-angiogenic pathways, and augment Th1 and CTL responses, and also has direct anti-tumor activity that is enhanced by TNFα. Thus, mediators induced by selective activation of TLR8 by VTX-2337 were assessed for direct activity on OVCAR5 tumor cells.

### TNFα mediating in part the interaction between TLR8 activation and PLD

Experiments were conducted to demonstrate that TNFα is responsible for the enhanced antitumor activity seen with the Doxil/VTX-2337 combination. Fresh elutriated human PBMCs were activated with either VTX-2337 (1 ug/ml) or anti CD3/28 beads and the media was collected after 6 hours. OVCAR5 cells were exposed to the culture media and an assessed of apoptosis was conducted at 24 hours, while cell viability was assessed at 48 hours.

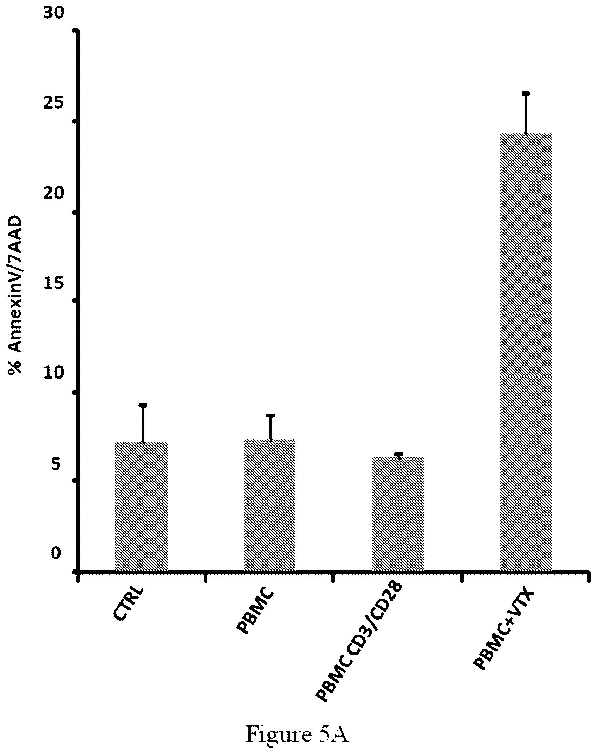

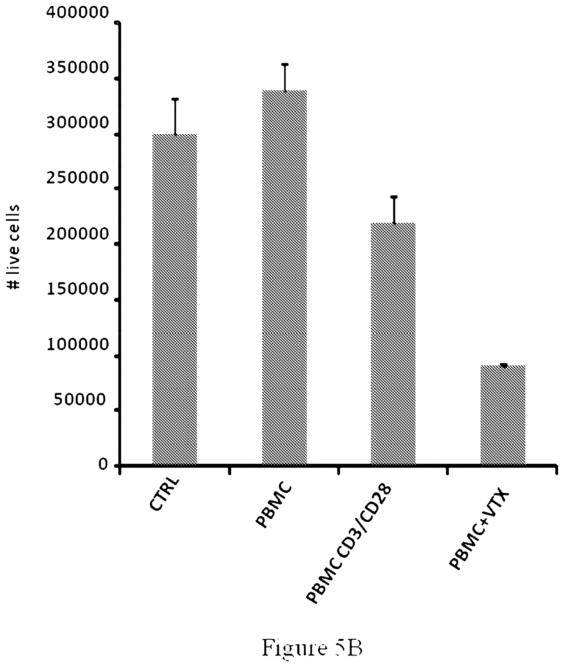

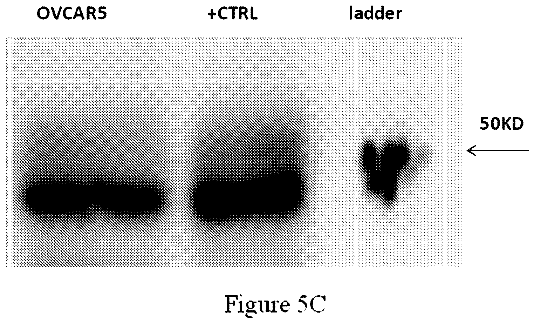

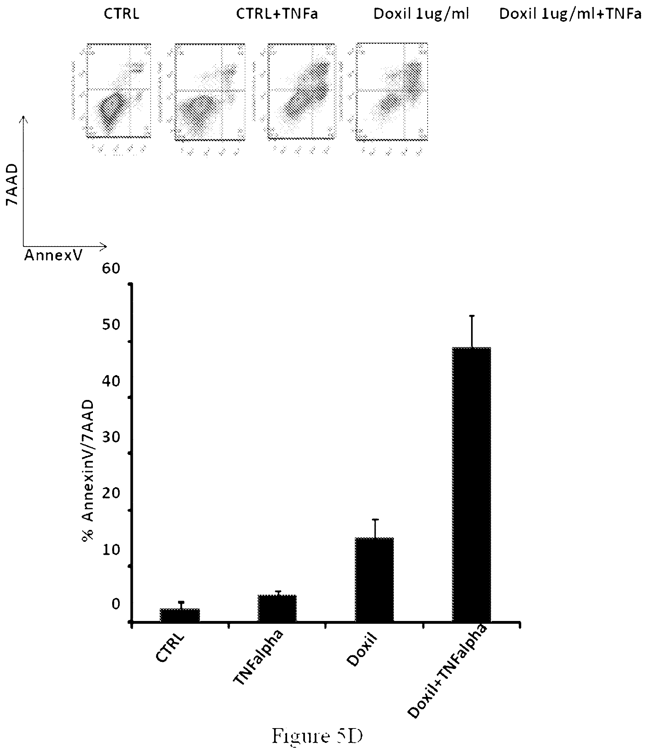

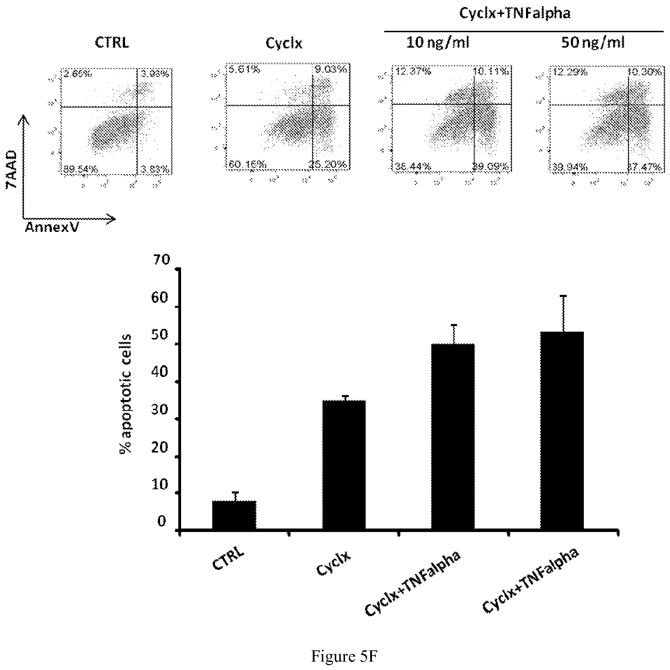

Besides tumor-specific CTLs, TLR8 activation may also invoke innate anti-tumor responses, including the release of soluble mediators such as members of the TNF family, which can act directly on tumor cells to induce apoptosis. Since TLR8 activation is associated with the production of high levels of TNFα, as shown in Figure 1, TNFα is a possible mediator of the effects of VTX-2337. Expression of the TNFα receptor 1 (TNFR1) in OVCAR5 cells was tested and documented by Western blot (Figure 5C). During the sensitivity test of OVCAR5 cells to TNFα, cells were incubated for 24 hours with 20 ng/ml of TNFα, a dose that exerts direct cytotoxic effects. Despite expressing TNFR1, OVCAR5 cells were resistant to TNFα-mediated apoptosis, as measured by annexin-V and 7AAD staining (Figure 5D).

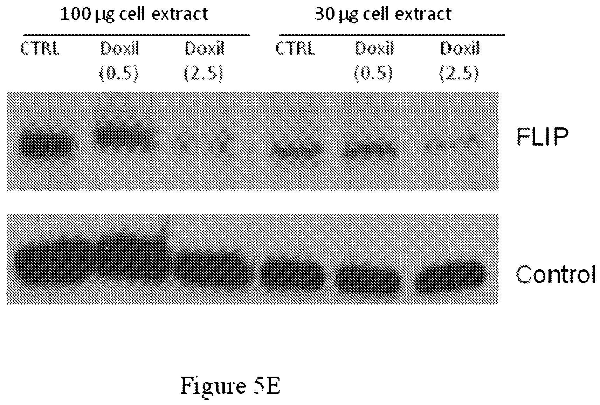

Tumor cells can resist TNFα-mediated apoptosis through overexpression of FADD-like IL-1h-converting enzyme (FLICE)-like inhibitory protein, or FLIP. FLIP, which can exist in both a long form (FLIP_{L}, 55 KDa) and a short form (FLIP_{S}, 28 KDa), can block apoptosis induced by TNFα family members including TNFα and TRAIL in different cell types. OVCAR5 cells expressed FLIP_{L}, the 55 Kd form of FLIP (Figure 5E, CTRL lane: control untreated cells).

Since doxorubicin triggers cell death and its activity in vivo is enhanced by VTX-2337, it has been tested that doxorubicin renders OVCAR5 cells more sensitive to TNFα-induced apoptosis. OVCAR5 cells were pre-incubated with either control media or media containing PLD at 1 µg/mL for 24h, then were incubated with either control media or media containing TNFα (20 ng/ml) for 12h. TNFα and Doxil alone induced minimal apoptosis, while a significant increase in apoptosis was detected when cells were treated with the combination of these two agents (Figure 5D). Treatment of OVCAR5 cells with PLD was found to inhibit FLIP expression, shown by western blot (Figure 5E).

TNFα was markedly up-regulated in vivo by VTX-2337 or combination treatment as shown in Figure 1C and Figure 2. The activation of the family of TNFα receptors on tumor cells can lead to activation of caspase 8, resulting in apoptosis. OVCAR5 cells were found to express the TNFα receptor 1, but were almost completely resistant to TNFα alone. However, an increase in apoptosis was detected when OVCAR5 cells were treated with combination agents in comparison to single agents. Doxorubicin kills cells by intercalation into DNA, which impairs DNA replication, inhibits translation leading to impaired macromolecular biosynthesis, and damages DNA though the production of ROS. It was also demonstrated inhibition of FLIP expression by OVCAR5 cells that were pretreated with PLD, suggesting that the impairment of new protein synthesis makes these tumor cells more sensitive to apoptosis mediated by TNF family members.

In summary, tumor cell death mediated by doxorubicin is not immunologically silent, but is mediated by activation of the immune system and the development of an adaptive immune response that participates in tumor cell control. However, the activity of doxorubicin can also impair the development of a protective immune response due to immune system toxicity. Unexpectedly, the addition of the TLR8 agonist VTX-2337 into the treatment regimen was found to enhance the anti-tumor effects of PLD^{®} in a novel ovarian cancer murine model using tumor-bearing NSG-HIS mice. VTX-2337 treatment was found to increase the migration of immune cells into tumors and enhance the development of tumor specific CTLs that were able to lyse OVCAR5 cells in vitro and control tumor growth in vivo. Activation of TLR8 also leads to the release of multiple mediators including TNFα, IL-12 and IFNγ that have anti-tumor activities and can further enhance the anti-tumor response. High levels of TNFα resulting from TLR8 activation can act directly on OVCAR5 cells to induce apoptosis, while the cells are made more sensitive to this apoptotic pathway due to the effects of doxorubicin on protein synthesis. Collectively, these results demonstrate that immunotherapy can increase the effectiveness of current cancer treatments in ovarian cancer. A study of VTX-2337 in combination with PLD^{®} as second-line treatment for patients with advanced recurrent ovarian cancer is ongoing.

### Example 2: Potency and selectivity of VTX-2337

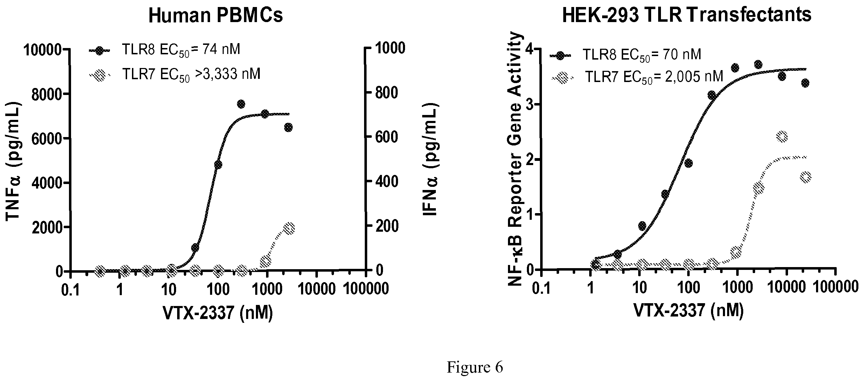

The half-maximal effective concentration (EC₅₀) for VTX-2337 activation of TLR8 and TLR7 was assessed in peripheral blood mononuclear cells (PBMCs) from 15 healthy donors and also in HEK293 cells transfected with TLR8 or TLR7 and an NF-κB driven reporter gene. As shown in Figure 6, in PBMCs, VTX-2337 stimulated TNFα production, a marker of TLR8 activation with an EC50 of 74 nM and IFNα production, a marker TLR7 activation, with an EC₅₀ >3,333 nM, indicating that VTX-2337 is > 45-fold more selective for TLR8 relative to TLR7. The data from the TLR7 and TLR8 HEK293 transfectants correlated closely to the data obtained using the PBMCs with EC₅₀s of 70 nM for TLR8 and 2,005 nM for TLR7. Also it was observed that VTX-2337 had no activity on TLR2, TLR3, TLR4, TLR5, TLR6, or TLR9 at concentrations up to 25 µM.

### Example 3: VTX-2337 stimulating a range of cytokines and chemokines in human whole blood

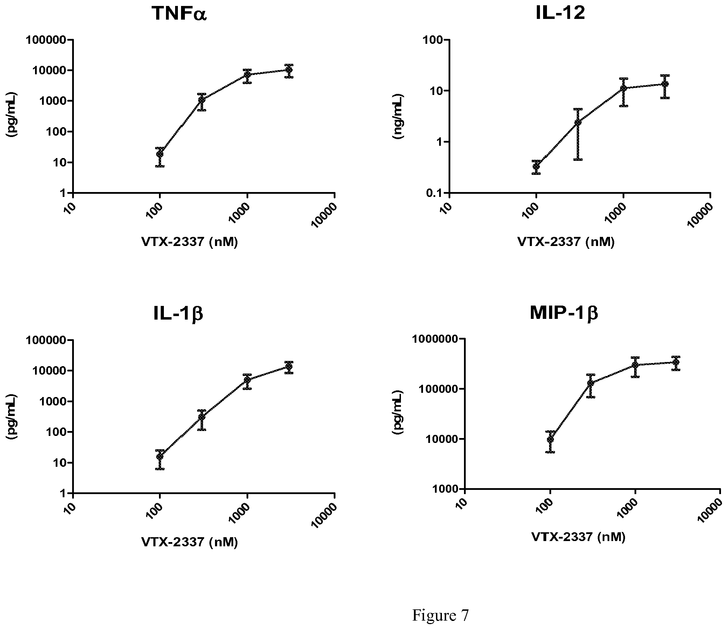

The immunostimulatory properties of VTX-2337 were characterized using the human multiple analyte panel (MAP), version 1.8 (Rules Based Medicine), to quantitate levels of 98 different analytes associated with inflammatory processes including cytokines, chemokines, and other proteins made by leukocytes in response to TLR7/8 activation. Whole blood was collected from 6 normal human volunteers and activated in vitro with VTX-2337 concentrations of 0.1, 0.3, 1.0 and 3.0 µM using the Instant Leukocyte Culture System. Co-culture with VTX-2337 resulted in dose dependent increases in a number of immune mediators including TNFα, IL-12p40, IL-1β, and MIP-1β, as shown in Figure 7.

### Example 4: VTX-2337 activating monocytes and myeloid dendritic cells (mDCs) but not plasmacytoid dendritic cells (pDCs)

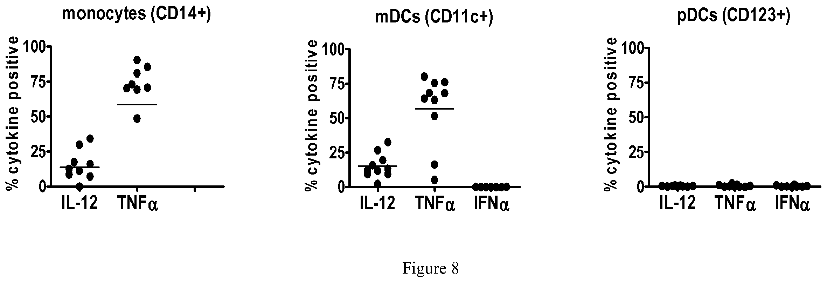

To evaluate the cellular specificity of VTX-2337, human PBMCs from healthy donors were stimulated with 0.8 µM VTX-2337 and the production intracellular cytokines in specific cell subsets present in PBMCs was assessed by flow cytometry. As shown in Figure 8, data are expressed as percentages of cells positive for IL-12, TNFα, and IFNα in monocytes (CD14+), pDC (CD123+), and mDC (CD11c+). Each data point represents the response from an individual donor (n=10). The horizontal bar represents the group mean. Intracellular levels of IL-12 and TNFα in were elevated in VTX-2337 treated monocytes and mDCs, but not pDCs, consistent with the cellular expression pattern of TLR8.

### Example 5: Phase I clinical study of VTX-2337

A dose escalation study was carried out to evaluate the safety, tolerability, and pharmacology of VTX-2337 when administered to adult subjects with advanced solid tumors or lymphoma. The primary objectives of the study were to assess the safety and pharmacokinetics of VTX-2337 and to identify any dose-limiting toxicities. Secondary objectives of the study were to assess the pharmacodynamic response to VTX-2337 and to determine the Maximum Tolerated Dose (MTD) for a single treatment cycle with VTX-2337.

**Study Procedures:** VTX-2337 was administered weekly via subcutaneous injection on Days 1, 8, and 15 of a 28-day dosing cycle for two cycles. Using a modified Fibonacci dose-escalation scheme, successive cohorts received doses ranging from 0.1 mg/m² to 3.9 mg/m² of VTX-2337. Plasma samples were collected for pharmacokinetic analysis after the first dose of the first treatment cycle and for pharmacodynamic analyses after the first dose of the first and second treatment cycles.

Clinical responses were assessed by RECIST and subjects with CR (i.e., complete response), PR (i.e., partial response), or SD (i.e., stable disease) were allowed to receive additional treatment cycles.

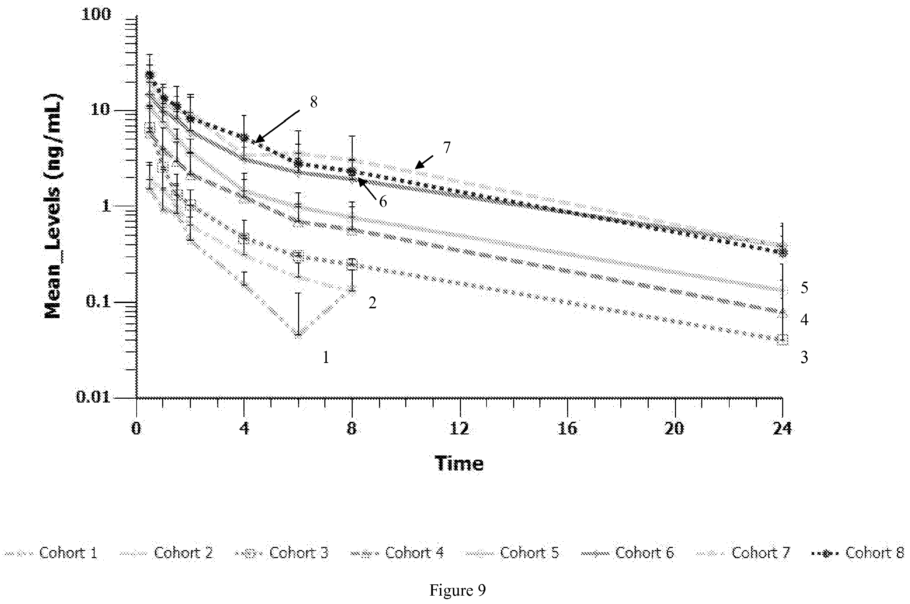

**Patient Demographics:** Thirty-three subjects with various late-stage solid malignancies were evaluated in 8 successive cohorts. The distribution of cancers evaluated was as follows: colorectal (n=9, or 27% of enrolled subjects), pancreatic (n=6 / 18%), melanoma (n=5 / 15%), cholangiocarcinoma (n=2 / 6%), renal cell (n=2 / 6%) and 1 subject each (3%) with hepatocellular, breast, endometrial, prostate, ovarian, adenoid cystic carcinoma of the tongue, metastatic basal cell, neuroendocrine carcinoma of the duodenum and liver, a tumor of unknown origin.
Samples were collected at 0.5, 1, 1.5, 2, 4, 8, and 24 hours after subcutaneous administration of the first dose of VTX-2337 and plasma levels of VTX-2337 were quantified by LC-MS/MS. VTX-2337 was rapidly absorbed into systemic circulation with the mean Tmax occurring between 0.5 and 0.8 hours following dosing. VTX-2337 was also cleared rapidly from circulation with a mean half-life (t_{1/2}) ranging between 1.7 and 6.7 hours. Peak plasma levels (Cₘₐₓ) and total systemic exposure both increased with increasing dose. Dose normalized (DN) values for Cₘₐₓ and AUQ_{(0-∞)} were calculated. In general, over the dose ranges evaluated, the pharmacokinetics of VTX-2337 appeared to be linear. The pharmacokinetic results are shown in Figure 9 and Table 1 below.

**Table 1**

| **Dose (mg/m²)** | **∼Dose (mg/kg)** | **T_{1/2} (hr)** | **Tₘₐₓ (hr)** | **Cₘₐₓ (ng/mL)** | **AUC_{(0-∞)} (ng•hr/mL)** | **DN Cₘₐₓ (ng/ml)/(mg/m²)** | **DN AUC_{(0-∞)} (ng•hr/mL)/(mg/m²)** |
|---|---|---|---|---|---|---|---|
| 0.1 | 0.0022 | 1.7 | 0.5 | 1.52 | 3.12 | 15.2 | 31.2 |
| 0.2 | 0.0054 | 3.0 | 0.8 | 1.97 | 4.74 | 9.9 | **23.7** |
| 0.4 | 0.011 | 6.6 | 0.5 | 6.69 | 10.94 | 16.7 | 27.4 |
| 0.8 | 0.022 | 5.3 | 0.5 | 5.93 | 19.17 | 7.4 | 24.0 |
| 1.3 | 0.035 | 5.6 | 0.5 | 10.9 | 29.50 | 8.4 | **22.7** |
| 2.0 | 0.054 | 6.7 | 0.5 | 14.6 | 59.15 | 7.3 | 29.6 |
| 2.8 | 0.076 | 5.7 | 0.5 | 19.9 | 80.35 | 7.1 | **28.7** |
| 3.9 | 0.105 | 5.3 | 0.5 | 23.0 | 81.10 | 5.9 | 20.8 |

To evaluate the pharmacodynamic (PD) response to VTX-2337, blood was collected at 0, 4, 8, 24 hours after subcutaneous administration of the first dose of the first cycle of VTX-2337. Plasma levels of immune mediators were quantified using the human multiple analyte panel (MAP), version 1.8 (Rules Based Medicine). Dose dependent increases in a number of biomarkers, including G-CSF, MCP-1, MIP-1β and TNFα, were observed between 4 and 8 hours post dosing with levels generally returning to baseline by 24 hours. Levels of mediators in plasma collected from 9 healthy volunteers are shown for comparison to the oncology population. Pharmacodynamic responses following subcutaneous administration of VTX-2337 are demonstrated in Table 2 below.

**Table 2**

| **Analyte** | **Time (Hr)** | **Normals** | **Cohort** 1 **(0.1 mg/m²)** | **Cohort** 2 **(0.2 mg/m²)** | **Cohort 3 (0.4 mg/m²)** | **Cohort 4 (0.8 mg/m²)** | **Cohort 5 (1.3mg/m²)** | **Cohort 6 (2.0 mg/m²)** | **Cohort** 7 **(2.8 mg/m²)** | **Cohort 8 (3.9 mg/m²)** |
|---|---|---|---|---|---|---|---|---|---|---|
| **G-CSF** | 0 | 0.7 ± 0.5 | 9.7 ± 1.9 | 7.8 ± 3.2 | 12.7 ± 6.0 | 13.2 ± 8.9 | 5.0 ± 0.3 | 9.9 ± 8.2 | 7.4 ± 3.2 | 29.1 ± 55.8 |
| **(pg/mL)** | 4 | | 8.6 ± 1.6 | 11.5 ± 7.8 | 9.5 ± 0.9 | 49.8 ± 80.3 | 20.5 ± 24.7 | 14.0 ± 4.3 | 12.4 ± 2.4 | 83.3 ± 104.7 |
| | 8 | | 11.9 ± 4.4 | 19.0 ± 18.5 | 63.8 ± 60.1 | 137 ± 124 | 196 ± 145 | 134 ± 149 | 553 ± 246 | 2151 ± 3586 |
| | 24 | | 9.2 ± 2.5 | 15.4 ± 9.0 | 15.8 ± 3.5 | 35.7 ± 14.7 | 30.8 ± 10.4 | 42.2 ± 31.6 | 59.0 ± 31.7 | 141.4 ± 228.8 |
| **MCP-1** | 0 | 131 ± 24.3 | 247 ± 62.7 | 245 ± 167 | 129 ± 30.0 | 260 ± 150 | 227 ± 79.8 | 265 ± 161 | 250 ± 110 | 249 ± 56 |
| **(pg/mL)** | 4 | | 223 ± 58.8 | 235 ± 84.8 | 137 ± 30.7 | 1037 ± 1920 | 911 ± 951 | 247 ± 80.5 | 719 ± 433 | 5227 ± 8362 |
| | 8 | | 280 ± 97.0 | 298 ± 100 | 251 ± 87.9 | 673 ± 386 | 954 ± 638 | 722 ± 224 | 2043 ± 760 | 8128 ± 10588 |
| | 24 | | 250 ± 85.0 | 301 ± 159 | 99.5 ± 6.9 | 267 ± 104 | 246 ± 158 | 320 ± 244 | 273 ± 109 | 532 ± 571 |
| **MIP-1β** | 0 | 136 ± 36.2 | 201 ± 73.8 | 230 ± 84.0 | 172 ± 59.0 | 165 ± 63.9 | 182 ± 36.9 | 136 ± 28.3 | 136 ± 22 | 236 ± 111 |
| **(pg/mL)** | 4 | | 196 ± 71.9 | 239 ± 74.0 | 215 ± 65.6 | 450 ± 641 | 543 ± 477 | 196 ± 62.5 | 517 ± 305 | 7793 ± 10824 |
| | 8 | | 204 ± 74.0 | 247 ± 84.7 | 213 ± 88.8 | 287 ± 177 | 688 ± 635 | 289 ± 111 | 646 ± 216 | 2259 ± 2456 |
| | 24 | | 207 ± 73.8 | 280 ± 115 | 173 ± 67.4 | 183 ± 55.9 | 228 ± 70.1 | 155 ± 26 | 152 **±** 82 | 276 ± 108 |
| **TNFα** | 0 | 5.2 ± 2.6 | 11.4 ± 4.4 | 9.7 ± 5.7 | 9.0 ± 4.9 | 16.7 ± 12.6 | 9.3 ± 4.1 | 4.4 ± 1.6 | 6.1 ± 1.8 | 14.9 ± 9.1 |
| **(pg/mL)** | 4 | | 10.5 ± 2.6 | 10.8 ± 5.0 | 10.8 ± 3.3 | 22.0 ± 14.6 | 14.9 ± 12.5 | 8.4 ± 1.5 | 10.9 **±** 1.4 | 58.9 ± 48.3 |
| | 8 | | 10.3 ± 4.0 | 10.5 ± 8.0 | 9.6 ± 3.1 | 19.0 ± 10.6 | 21.6 ± 13.3 | 10.0 ± 2.3 | 12.8 ± 2.5 | 42.7 ± 27.4 |
| | 24 | | 10.2 ± 3.6 | 12.4 ± 8.1 | 10.0 ± 2.9 | 17.5 ± 8.7 | 15.3 ± 4.2 | 10.0 ± 0.2 | 8.8 ± 4.1 | 24.2 ± 9.4 |

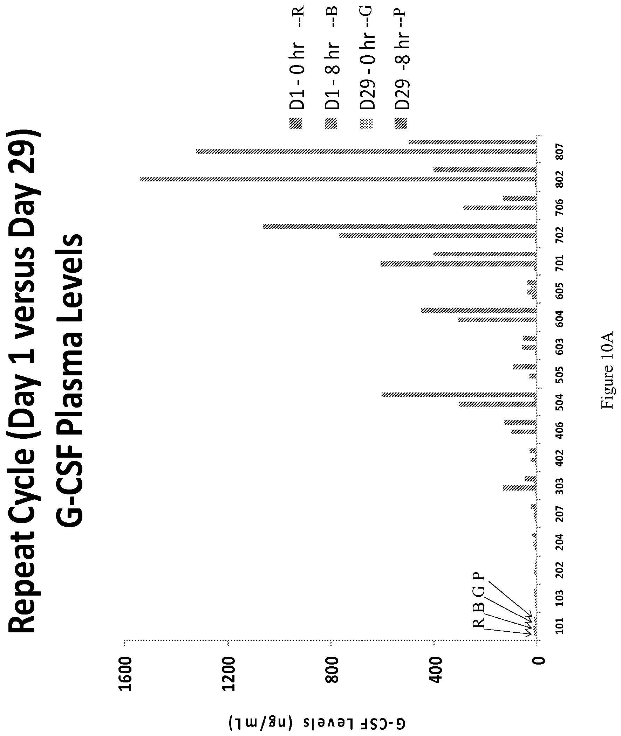

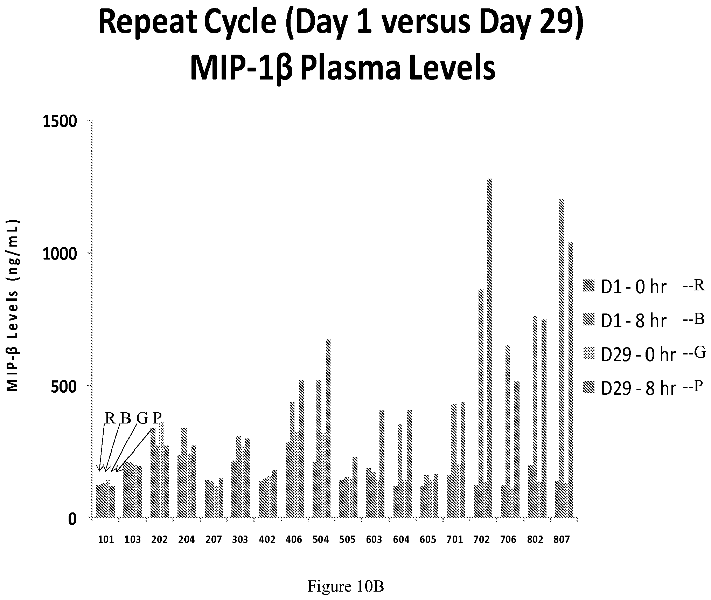

Pharmacodynamic responses to VTX-2337 were measured after the first dose of both the first and second treatment cycles with VTX-2337 (Day 1 and Day 29) to evaluate whether repeated administration would produce comparable effects. The bars in Figures 10A and 10B represent plasma levels of G-CSF and MIP-1β from individual patients in each cohort that received multiple cycles of VTX-2337. There was neither an augmentation nor broad desensitization of immune response after one treatment cycle with VTX-2337. In other words, pharmacodynamic responses are consistent over multiple treatment cycles.

**Adverse Event Profile:** VTX-2337 was generally safe and well tolerated. The most common drug related adverse events were injection site reactions, mild fevers and flu-like symptoms. These observations were not unexpected following administration of an immunomodulatory agent. No drug-related hematologic or gastrointestinal adverse events were observed.

In sum, it was observed that weekly subcutaneous administration of the novel TLR8 agonist VTX-2337 was generally safe and well tolerated; plasma levels of VTX-2337 and PD responses to VTX-2337 increased in a dose dependent manner; and that subcutaneous administration of VTX-2337 stimulated the production of multiple inflammatory mediators including cytokines and chemokines consistent with activation of an innate immune response.

### INCORPORATION BY REFERENCE

The entire disclosure of each of the patent documents and scientific articles referred to herein is incorporated by reference for all purposes.

### EQUIVALENTS

The invention can be embodied in other specific forms without departing from the spirit or essential characteristics thereof. The foregoing embodiments are therefore to be considered in all respects illustrative rather than limiting on the invention described herein. Scope of the invention is thus indicated by the appended claims rather than by the foregoing description, and all changes that come within the meaning and range of equivalency of the claims are intended to be embraced therein.

### Embodiments

1. A pharmaceutical composition for use in a combinational therapy of treating cancer comprising a formulation including a benzo[b]azepine TLR8 agonist and a pharmaceutically acceptable carrier, wherein the combinational therapy further comprises one or more additional treatment modalities.
2. The pharmaceutical composition of embodiment 1, wherein the TLR8 agonist is 2-amino-N,N-dipropyl-8-(4-(pyrrolidine-1-carbonyl)phenyl)-3H-benzo[b]azepine-4-carboxamide or a pharmaceutically acceptable salt thereof.
3. The pharmaceutical composition of embodiment 1 or 2, wherein the cancer is a solid cancer selected from the group consisting of ovarian cancer, breast cancer, head and neck cancer, renal cancer, bladder cancer, hepatocellular cancer, colorectal cancer, lymphoma, and any combination thereof.
4. The pharmaceutical composition of any of embodiments 1-3, wherein the one or more additional treatment modalities comprise administering an effective amount of an anti-cancer agent.
5. The pharmaceutical composition of any of embodiments 1-4, wherein the anti-cancer agent is doxorubicin, gemcitabine, cyclophosphamide, or a pharmaceutically acceptable salt thereof.
6. The pharmaceutical composition of any of embodiments 1-5, wherein the TLR8 agonist and the anti-cancer agent are administered concurrently or sequentially.
7. The pharmaceutical composition of any of embodiments 1-5, wherein the TLR8 agonist and the anti-cancer agent are administered separately by 5 days.
8. The pharmaceutical composition of embodiment 1 or 2, wherein the cancer is ovarian cancer, and the anti-cancer agent is a pegylated liposomal form of doxorubicin.
9. The pharmaceutical composition of embodiment 8, wherein the doxorubicin is administered intravenously.
10. The pharmaceutical composition of embodiment 1 or 2, wherein the cancer is breast cancer, and the anti-cancer agent is gemcitabine or cyclophosphamide.
11. The pharmaceutical composition of any of embodiments 1-10, wherein the subject is a mammal.
12. The pharmaceutical composition of any of embodiments 1-11, wherein the formulation is injected by a subcutaneous route, an intravenous route, an intramuscular route, or transdermal route.
13. The pharmaceutical composition of any of embodiments 1-11, wherein the formulation is administered subcutaneously.
14. The pharmaceutical composition of any of embodiments 1-13, wherein the one or more additional treatment modalities are selected from a chemotherapeutic agent, a cytokine, an antibody, a hormonal therapy, and a radiation therapy.
15. The pharmaceutical composition of any of embodiments 1-14, wherein the TLR8 agonist is dosed at a concentration from about 0.02 to about 10 mg/kg or from about 0.04 to about 5 mg/kg body weight of the subject.
16. The pharmaceutical composition of any of embodiments 1-14, wherein the TLR8 agonist is dosed at a concentration of about 0.02 mg/kg, about 0.05 mg/kg, about 0.075 mg/kg, about 0.1 mg/ kg, about 0.5 mg/kg, about 1 mg/kg, about 2 mg/kg, or about 5 mg/kg body weight of the subject.
17. The pharmaceutical composition of any of embodiments 1-14, wherein the TLR8 agonist is dosed at a concentration of from about 2.5 to 3.5 mg/m² of body surface area of the subject.
18. The pharmaceutical composition of any of embodiments 1-14, wherein the TLR8 agonist is dosed at a concentration of between 0.002 mg/kg and 0.006 mg/kg body weight of the subject.
19. The pharmaceutical composition of any of embodimentss 1-18, wherein the TLR8 agonist is administered to the subject on a weekly or biweekly basis.
20. The pharmaceutical composition of any of embodiments 4-9 and 11-19, wherein doxorubicin is administered at a dose of from about 0.02 to 10 mg/kg of body weight or from about 0.04 to 5 mg/kg of body weight of the subject.
21. The pharmaceutical composition of any of embodiments 1-20, wherein the formulation further comprises about from 1 % to about 30%, from about 5% to 15%, or from about 5% to about10% weight/volume of a cyclodextrin.
22. The pharmaceutical composition of any of embodiments 1-20, wherein the formulation further comprises about 1 %, about 5%, about 10%, about 15%, about 20%, about 25%, or about 30% weight/volume of a cyclodextrin.
23. The pharmaceutical composition of any of embodiments 1-20, wherein the formulation further comprises about 15% w /v of a cyclodextrin.
24. The pharmaceutical composition of any of embodiments 21-23, wherein the cyclodextrin is a beta-cyclodextrin.
25. The pharmaceutical composition of embodiment 1, wherein the cancer is lymphoma, and the one or more treatment modalities comprises a radiation therapy.
26. The pharmaceutical composition of embodiment 25, wherein the lymphoma is Non-Hodgkin's lymphoma.
27. A pharmaceutical composition for treating cancer comprising a benzo[b]azepine TLR8 agonist at a dose between 0.002 mg/kg/week and 0.006 mg/kg/week.
28. The pharmaceutical composition of embodiment 27, wherein the benzo[b]azepine TLR8 agonist is 2-amino-N,N-dipropyl-8-(4-(pyrrolidine-1-carbonyl)phenyl)-3H-benzo[b]azepine-4-carboxamide.
29. The pharmaceutical composition of embodiment 27, wherein the TLR8 agonist is 2-amino-N,N-dipropyl-8-(4-(pyrrolidine-1-carbonyl)phenyl)-3H-benzo[b]azepine-4-carboxamide or a pharmaceutically acceptable salt thereof.
30. A pharmaceutical composition comprising an anti-cancer agent and a formulation of a benzo[b]azepine TLR8 agonist.
31. The pharmaceutical composition of embodiment 30, wherein the TLR8 agonist is 2-amino-N,N-dipropyl-8-(4-(pyrrolidine-1-carbonyl)phenyl)-3H-benzo[b]azepine-4-carboxamide or a pharmaceutically acceptable salt thereof.
32. The pharmaceutical composition of embodiment 30, wherein the anti-cancer agent is doxorubicin, gemcitabine, cyclophosphamide, or a pharmaceutically acceptable salt thereof.
33. The pharmaceutical composition of embodiment 32, wherein doxorubicin is in a pegylated liposomal form.
34. The pharmaceutical composition of embodiment 30, wherein the formulation has a concentration from about 0.01 to 50 mg/ml of the TLR8 agonist.
35. The pharmaceutical composition of embodiment30, wherein the formulation has a concentration from about 0.5 mg/ml to about 50 mg/ml, from about 1 mg/ml to about 40 mg/ml, or from about 2 mg/ml to about 15 mg/ml of the TLR8 agonist.
36. The pharmaceutical composition of embodiment 30, wherein the formulation has a concentration from about 0.5 mg/ml to about 10 mg/ml, from about 0.5 mg/ml to about 8 mg/ml, from about 0.5 mg/ml to about 15 mg/ml, from about 0.5 mg/ml to about 4 mg/ml, or from about 0.5 mg/ml to about 2 mg/ml of the TLR8 agonist.
37. The pharmaceutical composition of embodiment 30, wherein the formulation has a concentration of about 0.01 mg/ml, about 0.5 mg/ml, about 1 mg/ml, about 2 mg/ml, about 4 mg/ ml, about 15 mg/ml, about 8 mg/ml, about 10 mg/ml, about 15 mg/ml, about 20 mg/ml, about 25 mg/ml, about 30 mg/ml, about 40 mg/ml, or about 50 mg/ml of the TLR8 agonist.
38. The pharmaceutical composition of embodiment 30, wherein the formulation further comprises about from 1 % to about 30%, from about 5% to 15%, or from about 5% to about 10% weight/volume of a cyclodextrin.
39. The pharmaceutical composition of embodiment 30, wherein the formulation further comprises about 1 %, about 5%, about 10%, about 15%, about 20%, about 25%, or about 30% weight/volume of a cyclodextrin.
40. The pharmaceutical composition of embodiment 30, wherein the formulation further comprises about 15% w/v of a cyclodextrin.
41. The pharmaceutical composition of any of embodiments 38-40, wherein the cyclodextrin is a beta-cyclodextrin.
42. The pharmaceutical composition of embodiment 41, wherein the beta-cyclodextrin is sulfobutyl ether beta-cyclodextrin.
43. A pharmaceutical pack or kit comprising one or more containers filled with a liquid or lyophilized form a pharmaceutical composition of any of embodiments 30-42.
44. The pack or kit of embodiment 43, wherein the pharmaceutical composition is in aqueous formulation.
45. A pharmaceutical pack or kit comprising one or more first containers filled with a liquid or lyophilized form a formulation of a benzo[b]azepine TLR8 agonist and one or more second containers filled with an anti-cancer agent.
46. The pack or kit of embodiment 45, wherein the TLR8 agonist is 2-amino-N,N-dipropyl-8-(4-(pyrrolidine-1-carbonyl)phenyl)-3H-benzo[b]azepine-4-carboxamide or a pharmaceutically acceptable salt thereof.
47. The pack or kit of embodiment 45, wherein the anti-cancer agent is doxorubicin, gemcitabine, cyclophosphamide, or a pharmaceutically acceptable salt thereof.

## Claims

1. A pharmaceutical composition for use in a combinational therapy of treating cancer in a subject, preferably a mammal, comprising a formulation including a benzo[b]azepine TLR8 agonist and a pharmaceutically acceptable carrier, wherein the combinational therapy further comprises one or more additional treatment modalities.

2. The pharmaceutical composition for use of claim 1, wherein the TLR8 agonist is 2-amino-N,N-dipropyl-8-(4-(pyrrolidine-1-carbonyl)phenyl)-3H-benzo[b]azepine-4-carboxamide or a pharmaceutically acceptable salt thereof.

3. The pharmaceutical composition for use of claim 1 or 2, wherein the cancer is a solid cancer selected from the group consisting of ovarian cancer, breast cancer, head and neck cancer, renal cancer, bladder cancer, hepatocellular cancer, colorectal cancer, lymphoma, and any combination thereof.

4. The pharmaceutical composition for use of any one of claims 1-3, wherein the one or more additional treatment modalities comprise administering an effective amount of an anti-cancer agent, preferably selected from doxorubicin, gemcitabine, cyclophosphamide, and a pharmaceutically acceptable salt thereof.

5. The pharmaceutical composition for use of claim 4, wherein (i) the formulation and the anti-cancer agent are administered concurrently; or (ii) the formulation and the anti-cancer agent are administered sequentially, preferably separated by 5 days, and wherein the formulation and anti-cancer agent are administered in one cycle or more than one cycle.

6. The pharmaceutical composition for use of any one of claims 1-3, wherein the one or more additional treatment modalities are selected from a chemotherapeutic agent, a cytokine, an antibody, a hormonal therapy, and a radiation therapy.

7. The pharmaceutical composition for use of any one of claims 4-6, wherein (i) the cancer is ovarian cancer, and the anti-cancer agent is a pegylated liposomal form of doxorubicin; (ii) the cancer is breast cancer, and the anti-cancer agent is gemcitabine or cyclophosphamide; (iii) the cancer is a head and neck cancer, and the doxorubicin is in a pegylated liposomal form; or (iv) the cancer is lymphoma, preferably Non-Hodgkin's lymphoma, and the one or more treatment modalities comprises a radiation therapy.

8. The pharmaceutical composition for use of any one of claims 1-7, wherein the formulation is injected by a subcutaneous route, an intravenous route, an intramuscular route, or a transdermal route, preferably by a subcutaneous route.

9. The pharmaceutical composition for use of any one of claims 1-8, wherein the formulation is dosed to provide the TLR8 agonist at a concentration from about 0.02 to about 10 mg/kg, or from about 0.04 to about 5 mg/kg body weight of the subject, preferably the formulation is administered to the subject on a weekly or biweekly basis, or below 0.007 mg/kg/week or between 0.002 mg/kg/week to 0.006 mg/kg/week.

10. The pharmaceutical composition for use of any one of claims 1-8, wherein the formulation is dosed to provide the TLR8 agonist at a concentration of from about 0.1 to about 10 mg/m² or from about 2.5 to 3.5 mg/m² of body surface area of the subject, preferably the formulation is administered to the subject on a weekly or biweekly basis.

11. The pharmaceutical composition for use of any one of claims 4-5 and 7-10, wherein the doxorubicin is in a form suitable for intravenous administration, preferably in a pegylated liposomal form.

12. The pharmaceutical composition for use of any one of claims 4-5 and 7-11, wherein the doxorubicin is administered at a dose of from about 0.02 to 10 mg/kg of body weight, from about 0.04 to 5 mg/kg of body weight of the subject or not more than 50 mg/m² of the body surface area of the subject.

13. The pharmaceutical composition for use of any one of claims 1-12, wherein the formulation further comprises from about 1% to about 30%, from about 5% to 15%, or from about 5% to about10% weight/volume of a cyclodextrin, preferably a beta-cyclodextrin, and more preferably a sulfobutyl ether β-cyclodextrin.

14. A pharmaceutical pack or kit comprising one or more first containers filled with a liquid or lyophilized form of a formulation of a benzo[b]azepine TLR8 agonist and one or more second containers filled with an anti-cancer agent, preferably the TLR8 agonist is 2-amino-N,N-dipropyl-8-(4-(pyrrolidine-1-carbonyl)phenyl)-3H-benzo[b]azepine-4-carboxamide or a pharmaceutically acceptable salt thereof.

15. The pharmaceutical pack or kit of claim 14, wherein the anti-cancer agent is doxorubicin, gemcitabine, cyclophosphamide, or a pharmaceutically acceptable salt thereof.
